# EUROPEAN PATENT SPECIFICATION

(11) **EP 0 010 560 B1**
(45) Date of publication and mention of the grant of the patent: **13.04.1983**
(21) Application number: 78300579.6
(22) Date of filing: 01.11.1978
(51) Int. Cl.: C07D 251/52, C07D 251/16, C07D 239/48, C07D 409/12, A01N 47/36

(54) **Herbicidal sulfonamides, their preparation, compositions containing them and use thereof**
Herbizide Sulfonamide, ihre Herstellung, sie enthaltende Zusammensetzungen und ihre Verwendung
Sulfonamides à activité herbicide, leur préparation, compositions les contenant et leur utilisation

(43) Date of publication of application: 14.05.1980
(73) Proprietor: E.I. DU PONT DE NEMOURS AND COMPANY, Wilmington Delaware 19898 (US)
(72) Inventor: Levitt, George, Cardiff Wilmington, Delaware 19810 (US)
(74) Representative: Hildyard, Edward Martin

## Description

This invention relates to herbicidal sulfonamides, and the preparation and use thereof in agriculture.

French Patent No. 1,468,747 discloses the following para-substituted phenylsulfonamides, useful as antidiabetic agents: 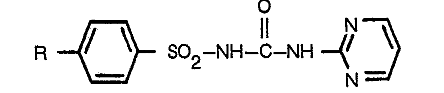 where R = H, halogen, CF₃ or alkyl.

Logemann et al., Chem. Ab., 53, 18052 g (1959), disclose a number of sulfonamides, including uracil derivatives and those having the formula: 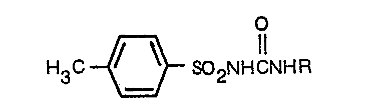 wherein R is butyl, phenyl or 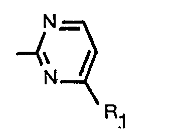 and Rᵢ is hydrogen or methyl. When tested for hypoglycemic effect in rats (oral doses of 25 mg/100 g), the compounds in which R is butyl or phenyl were most potent. The others were of low potency or inactive.

Our DE-A-2,715,786 discloses inter alia herbicidal sulfonamides of the formula 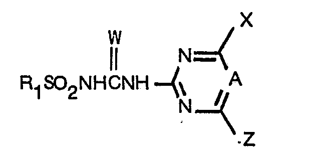 wherein W is 0 or S; Rᵢ can be optionally substituted phenyl or thienyl; X is H, Cl, Br, CH₃, C₂H₅, alkoxy of 1-3 carbon atoms, trifluoromethyl, CH₃S- or CH₃0CH₂₋; and Z is methyl or methoxy. The X-substituents are entirely distinct from those envisaged by the present invention.

Wojciechowski, J. Acta. Polon. Pharm. 19, p. 121-5 (1962) [Chem. Ab., 59 1633 e] describes the synthesis of N-[(2,6-dimethoxypyrimidin-4-yl]aminocarbonyl]-4-methylbenzenesulfonamide: 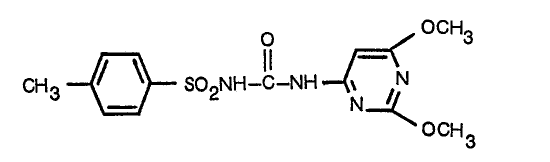 Based upon similarity to a known compound, the author predicted hypoglycemic activity for the foregoing compound.

Netherlands Patent 121,788, published September 15, 1966, teaches the preparation of compounds of Formula (i), and their use as general or selective herbicides: 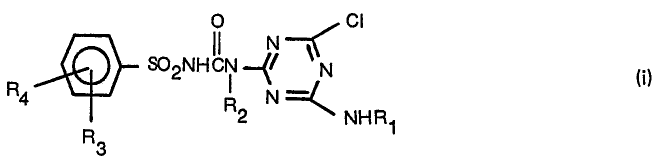 wherein
R, and R₂ may independently be alkyl of 1-4 carbon atoms; and
R₃ and R₄ may independently be hydrogen, chlorine or alkyl of 1-4 carbon atoms.

Compounds of Formula (ii), and their use as antidiabetic agents, are reported in J. Drug Res. 6, 123 (1974): 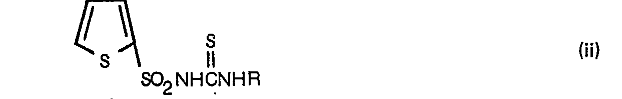 wherein R is pyridyl.

The presence of undesired vegetation causes substantial damage to useful crops, especially agricultural products that satisfy man's basic food needs, such as soybeans, wheat, and the like. The current population explosion and concomitant world food shortage demand improvements in the efficiency of producing these crops. Prevention or minimizing the loss of a portion of such valuable crops by killing, or inhibiting the growth of undesired vegetation is one way of improving this efficiency.

A wide variety of materials useful for killing, or inhibiting (controlling) the growth of undesired vegetation is available; such materials are commonly referred to as herbicides. The need exists, however, for still more effective herbicides that destroy or retard weeds without causing significant damage to useful crops.

According to this invention, there are provided compounds of Formula I and their agriculturally suitable salts, suitable agricultural compositions containing them, and methods of using them as selective, as well as general herbicides having both preemergence and postemergence activity. These compounds are highly active herbicides. They are especially useful for controlling weeds in wheat. 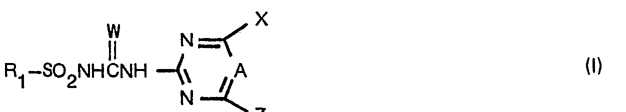 where 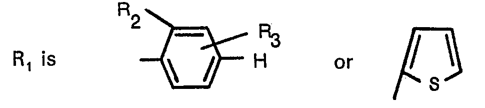
R₂ and R₃ are independently hydrogen, fluorine, chlorine, bromine, methyl, methoxy, nitro or trifluoromethyl;
W is oxygen or sulfur;
X is -NHCH₃ or ―N(CH₃)₂;
Z is methyl or methoxy; and
A is C or N. H

and their agriculturally suitable salts, provided that when R₂ is nitro or trifluoromethyl, R₃ cannot be nitro or trifluoromethyl.

Preferred for their high herbicidal activity or favorable cost or both are those compounds of Formula I where independently: 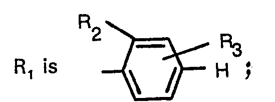
R₂ is fluorine, chlorine, bromine, methyl or nitro; and
R₃ is hydrogen, fluorine, chlorine, bromine, methyl or nitro.

More preferred for their higher herbicidal activity or more favorable cost or both are those compounds of Formula I where: 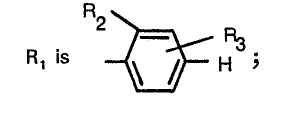
R₂ is chlorine, methyl or nitro; and
R₃ is hydrogen, chlorine or methyl.

Most preferred for their excellent herbicidal activity or more favorable cost or both are those compounds of Formula I where: 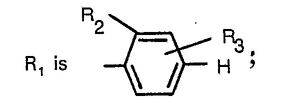
R₂ is chlorine, methyl or nitro;
R₃ is hydrogen, chlorine or methyl; and
W is oxygen.

Specifically preferred for their outstanding herbicidal activity or highly favorable cost or both are:
1. 2-chloro-N-[(4-methoxy-6-methylamino-1,3,5-triazin-2-yl)aminocarbonyl]benzenesulfonamide, m.p. 224-228°C;
2. 2-chloro-N-[(4-dimethylamino-6-methoxy-1,3,5-triazin-2-yl)aminocarbonyl]benzenesulfonamide, m.p. 200-206°C;
3. N-[(4-dimethylamino-6-methoxy-1,3,5-triazin-2-yl)aminocarbonyl]-2-methylbenzenesulfon- amide, m.p. 194-198°C;
4. N-[(4-methoxy-6-methylamino-1,3,5-triazin-2-yl)aminocarbonyl]-2-methylbenzenesulfon- amide, m.p. 199-202°C;
5. N-[(4-dimethylamino-6-methoxypyrimidin-2-yl)aminocarbonyl]-2-methylbenzenesulfonamide, m.p. 238-239°C;
6. N-[(4-dimethylamino-6-methoxy-1,3,5-triazin-2-yl)aminocarbonyl]-2-nitrobenzenesulfonamide, m.p. 212-213°C; and
7. N-[(4-methylamino-6-methoxy-1,3,5-triazin-2-yl)aminocarbonyl]-2-nitrobenzenesulfonamide.

In addition to having excellent activity for broad spectrum control of vegetation, the compounds of Formula I are also useful for selective control of volunteer corn in soybeans, weeds in wheat, brush control and water hyacinth control. Moreover, the compounds of Formula I are useful plant growth regulants, e.g. increasing sugar content in sugar cane and sorghum and suppressing seed head formation in grasses such as Bahia grass.

As shown in Equation I, the compounds of Formula I can be prepared by combining an appropriate 2-aminoheterocycle of Formula III with an appropriately substituted sulfonyl isocyanate or isothiocyanate of Formula II; R,, A, W, X and Z being as previously defined.

### Equation l

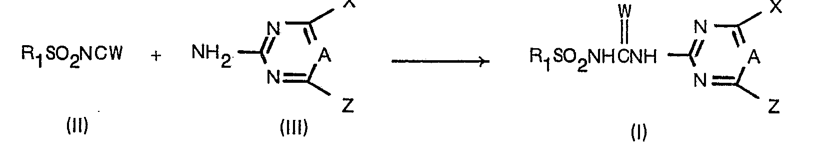

The reaction is best carried out in inert aprotic organic solvents such as methylene chloride, tetrahydrofuran or acetonitrile, at ambient pressure and temperature. The mode of addition is not critical; however, it is often convenient to add the sulfonyl isocyanate or isothiocyanate to a stirred suspension of the aminoheterocycle. Since isocyanates and isothiocyanates usually are liquids, their addition is more easily controlled.

The reaction is generally exothermic. In some cases, the desired product is insoluble in the warm reaction medium and crystallizes from it in pure form. Products soluble in the reaction medium are isolated by evaporation of the solvent, trituration of the solid residue with solvents such as 1-chlorobutane, ethyl ether, or pentane, and filtration.

The intermediate sulfonyl isocyanates of Formula II (wherein W is 0) can be prepared by reacting corresponding sulfonamides with phosgene in the presence of n-butyl isocyanate at reflux in a solvent such as chlorobenezene, according to the procedure of H. Ulrich and A. A. Y. Sayigh, Newer Methods of Preparative Organic Chemistry, Vol. VI, p. 223-241, Academic Press, New York and London, W. Foerst, Ed. In cases where formation of the desired sulfonyl isocyanate is difficult by the above procedure, the sulfonyl urea formed by the reaction of butyl isocyanate with the appropriate sulfonamide is treated with phosgene according to the above reference.

The preparation of sulfonamides from ammonium hydroxide and sulfonyl chlorides is widely reported in the literature, e.g. Crossley et al., J. Am. Chem. Soc., 60, 2223 (1938).

Certain sulfonyl chlorides are best prepared by chlorosulfonation of a substituted benzene or thiophene according to the teaching of H. T. Clarke et al. Org. Synth., Coll. Vol. 1, 2nd Ed. 1941, p. 85. Other benzenesulfonyl chlorides are best made by diazotization of the appropriate aniline with sodium nitrite in HCI, followed by reaction of the diazonium salt with sulfur dioxide and cuprous chloride in acetic acid according to the teaching of H. L. Yale and F. Sowinski, J. Org. Chem. 25 1824 (1960).

Sulfonyl isothiocyanates can be prepared by treatment of sulfonamides with carbon disulfide and potassium hydroxide followed by reaction of the dipotassium salt with phosgene according to the teaching of K. Hartke, Arch. Pharm., 299, 174 (1966).

The synthesis of heterocyclic amine derivatives has been reviewed in "The Chemistry of Heterocyclic Compounds", a series published by Interscience Publ., New York and London. 2-Amino- pyrimidines are described by D. J. Brown in "The Pyrimidines", Vol. XVI of the above series.

2-Amino-1,3,5-triazines can be synthesized according to the methods described by E. M. Smolin and L. Rapaport in "s-Triazines and Derivatives", Vol. XIII, of the same series.

Agriculturally suitable salts of compounds of Formula I are also useful herbicides and can be prepared in a number of ways known to the art. For example, metal salts can be made by treating compounds of Formula I with a solution of an alkali or alkaline earth metal salt having a sufficiently basic anion (e.g. hydroxide, alkoxide, carbonate or hydride). Quarternary amine salts can be made by similar techniques.

Salts of compounds of Formula I can also be prepared by exchange of one cation for another. Cationic exchange can be effected by direct treatment of an aqueous solution of a salt of a compound of Formula I (e.g. alkali or quaternary amine salt) with a solution containing the cation to be exchanged. This method is most effective when the desired salt containing the exchanged cation is insoluble in water and can be separated by filtration.

Exchange may also be effected by passing an aqueous solution of a salt of a compound of Formula I (e.g. an alkali metal or quaternary amine salt) through a column packed with a cation exchange resin containing the cation to be exchanged. In this method, the cation of the resin is exchanged for that of the original salt and the desired product is eluted from the column. This method is particularly useful when the desired salt is water-soluble.

Acid addition salts, useful in this invention, can be obtained by reacting a compound of Formula I with a suitable acid, e.g. p-toluenesulfonic acid, trichloroacetic acid or the like.

The compounds of this invention and their preparation are further illustrated by the following examples wherein temperatures are given in degrees centigrade and parts are by weight unless otherwise designated.

### Example 1

### 2-Ch!oro-N-[(4-methoxy-6-methyiamino-1,3,5-triazin-2-yi)aminocarbonyi]benzenesuifonamide

To a solution of 2.1 g of 2-amino-4-methoxy-6-methylamino-1,3,5-triazine in 60 ml of hot acetonitrile was added, dropwise, 3.3 g of 2-chlorobenzenesulfonylisocyanate in 20 ml of acetonitrile. After stirring for 18 hours at room temperature, the mixture was filtered to yield the product named above melting at 224-228°.

By using the procedure of Example 1 with equivalent amounts of the appropriate amino-1,3,5-triazine derivative and sulfonyl isocyanate or sulfonylisothiocyanate, the compounds of Table 1 can be prepared.

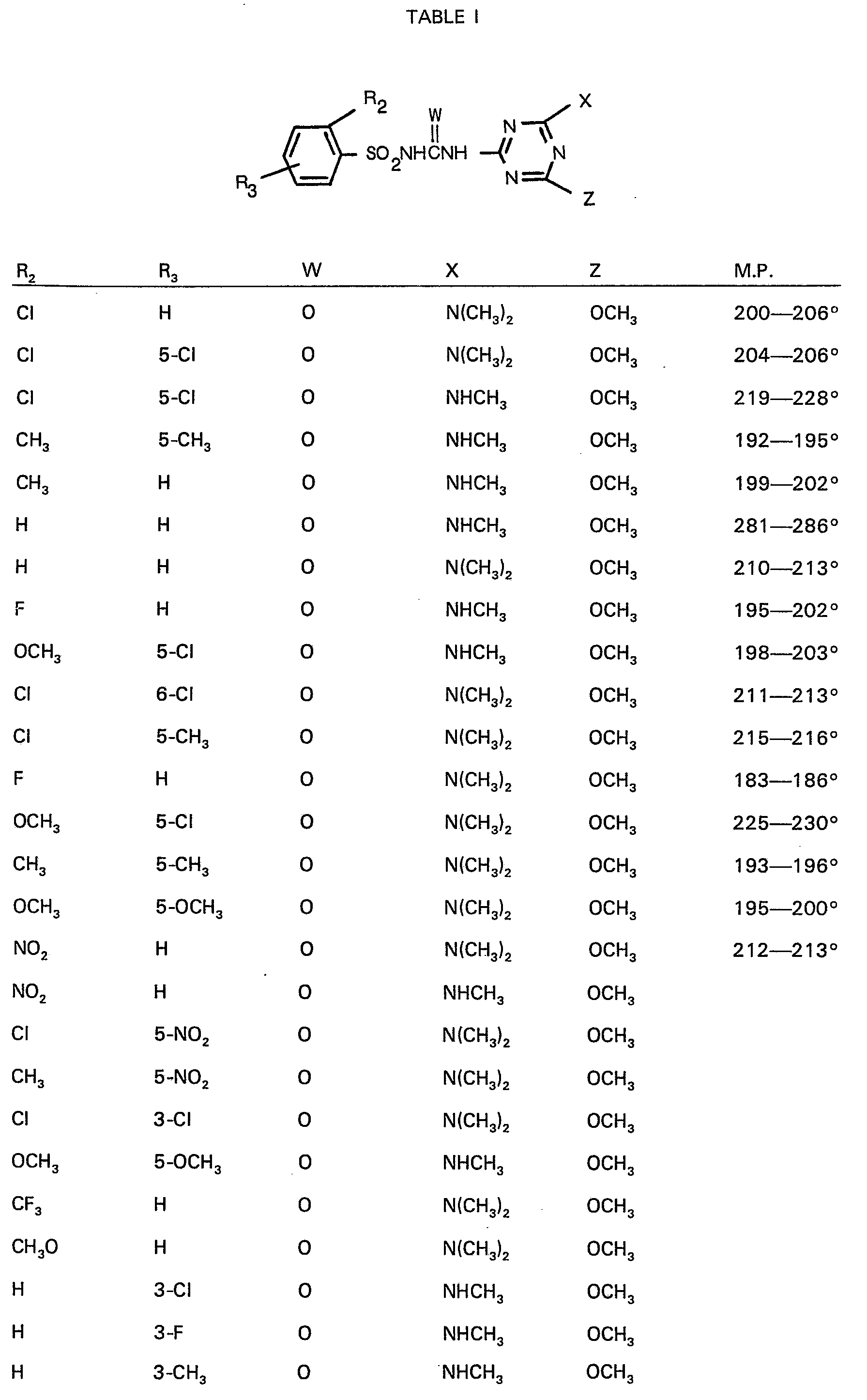

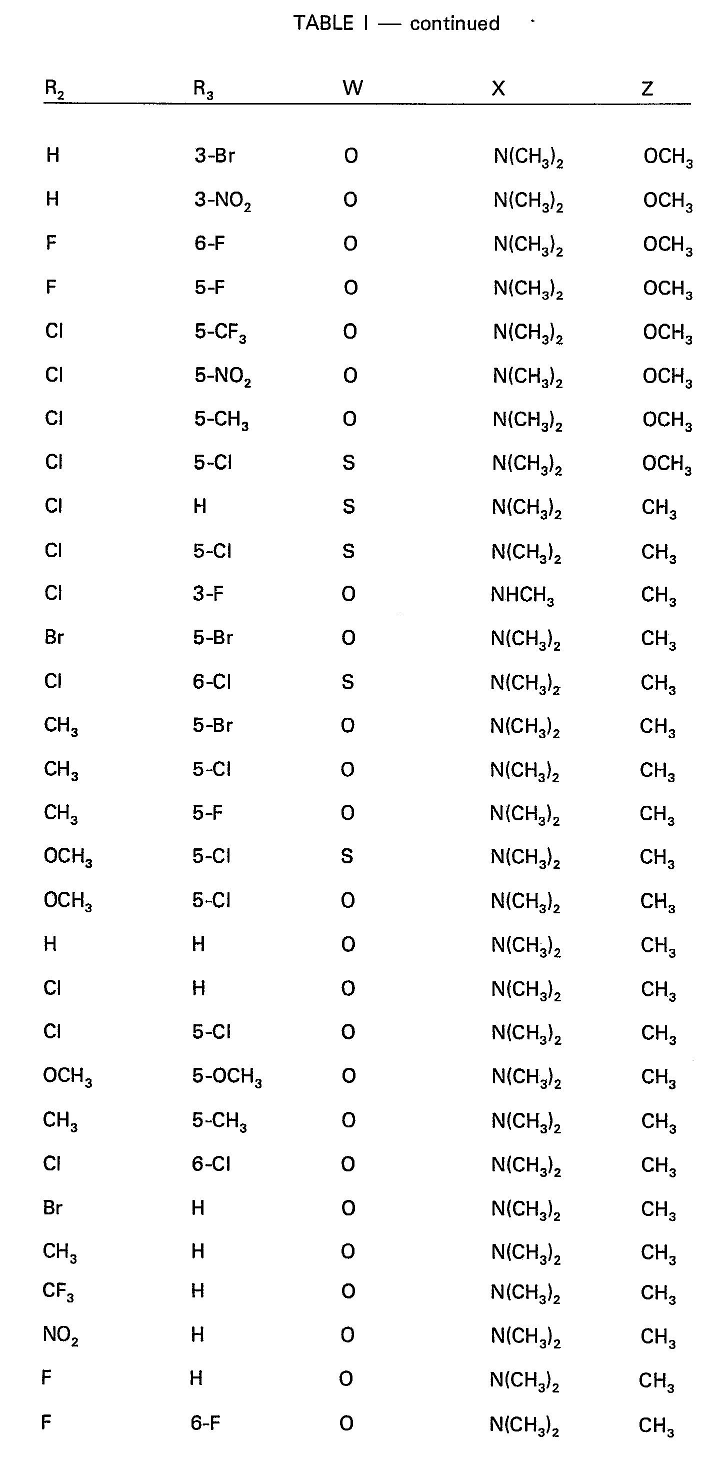

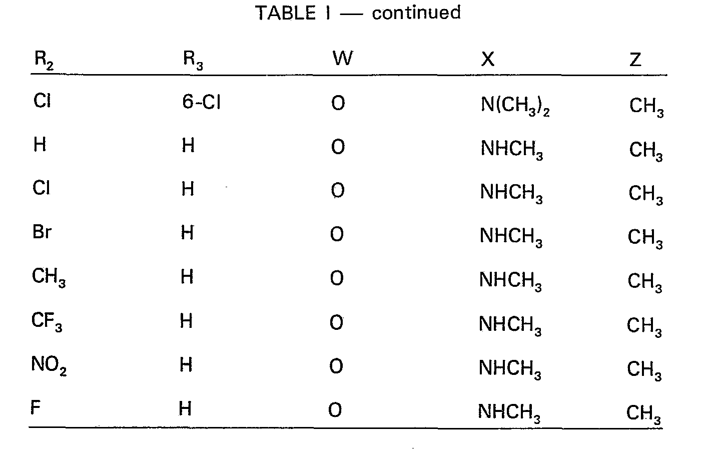

### Example 2

### N-[(4-Dimethylamino-6-methoxy-1,3,5-triazin-2-yl)aminocarbonyl]-2-methylbenzenesulfonamide

To a stirred solution of 0.85 g of 2-amino-4-dimethylamino-6-methoxy-1,3,5-triazine in 40 ml of methylene chloride was added, dropwise, 1.0 g of 2-methylbenzenesulfonyl isocyanate in 10 ml of methylene chloride. After stirring 24 hours, the resulting solution was evaporated to yield a solid. Recrystallization from benzene/hexane yielded the product named above melting at 194-198°.

### Example 3

### N-[(4-Dimethylamino-6-methoxy-1,3,5-triazin-2-yl)aminocarbonyl]-2-thiophenesulfonamide

To a stirred solution of 1.7 g of 2-amino-4-dimethylamino-6-methoxy-1,3,5-triazine in 50 ml of hot acetonitrile was added, dropwise, 1.9 g of thiophenesulfonyl isocyanate in 10 ml of acetonitrile. After stirring for 18 hours, the mixture was filtered to yield 2.0 g of the product named above melting at 192-195°.

By using this procedure of Example 3, N=[(4-methoxy-6-methylamino-1,3,5-triazin-2-yl)aminocarbonyl]-2-thiophenesulfonamide was prepared melting at 208-210°.

### Example 4

### N-[(4-Methoxy-6-methylamino-1,3,5-triazin-2-y()aminocarbonyl]-2-thiophenesulfonamide, sodium salt

To a slurry of 2.5 g of N-[(4-methoxy-6-methylamino-1 ,3,5-triazin-2-yl)aminocarbonyl]-2-thiophenesulfonamide and 50 ml of water was added 3 ml of 10% sodium hydroxide. The resulting solution was filtered and after cooling the filtrate, a white crystalline solid formed. Filtration afforded 1.1 g of the product named above melting at 297-298°.

### Example 5

### N-[(4-Dimethylamino-6-methylpyrimidin-2-yl)aminocarbonyl]benzenesulfonamide

To a suspension of 15.2 g of 2-amino-4-dimethylamino-6-methylpyrimidine in 400 ml of methylene chloride at ambient temperature was added slowly 18.3 g of benzenesulfonylisocyanate. After stirring for four hours the mixture was stripped in-vacuo and the white semisolid residue triturated with ethyl ether and isolated by filtration. The solid was then slurried in hot acetone, cooled and refiltered to yield the solid named above which decomposed at 180-182^{1.}

### Example 6

### 2-Chloro-N-[(4-dimethylamino-6-methoxypyrimidin-2-yl)-aminocarbonyl]benzenesulfonamide

To 700 ml of acetonitrile containing 25 g of 2-amino-4-dimethylamino-6-methoxypyrimidine at ambient temperature was added, dropwise, 32.5 g of 2-chlorobenzenesulfonylisocyanate. The mixture was warmed to 40° and then allowed to stir for five hours at ambient temperature. The solid product named above was isolated by filtration and washed with small amount of cold ethyl ether. It melted at 224-226°.

By using the procedure of Example 6 with equivalent amounts of the appropriate 2-amino- pyrimidine derivative and sulfonylisocyanate, the compounds of Table II can be prepared.

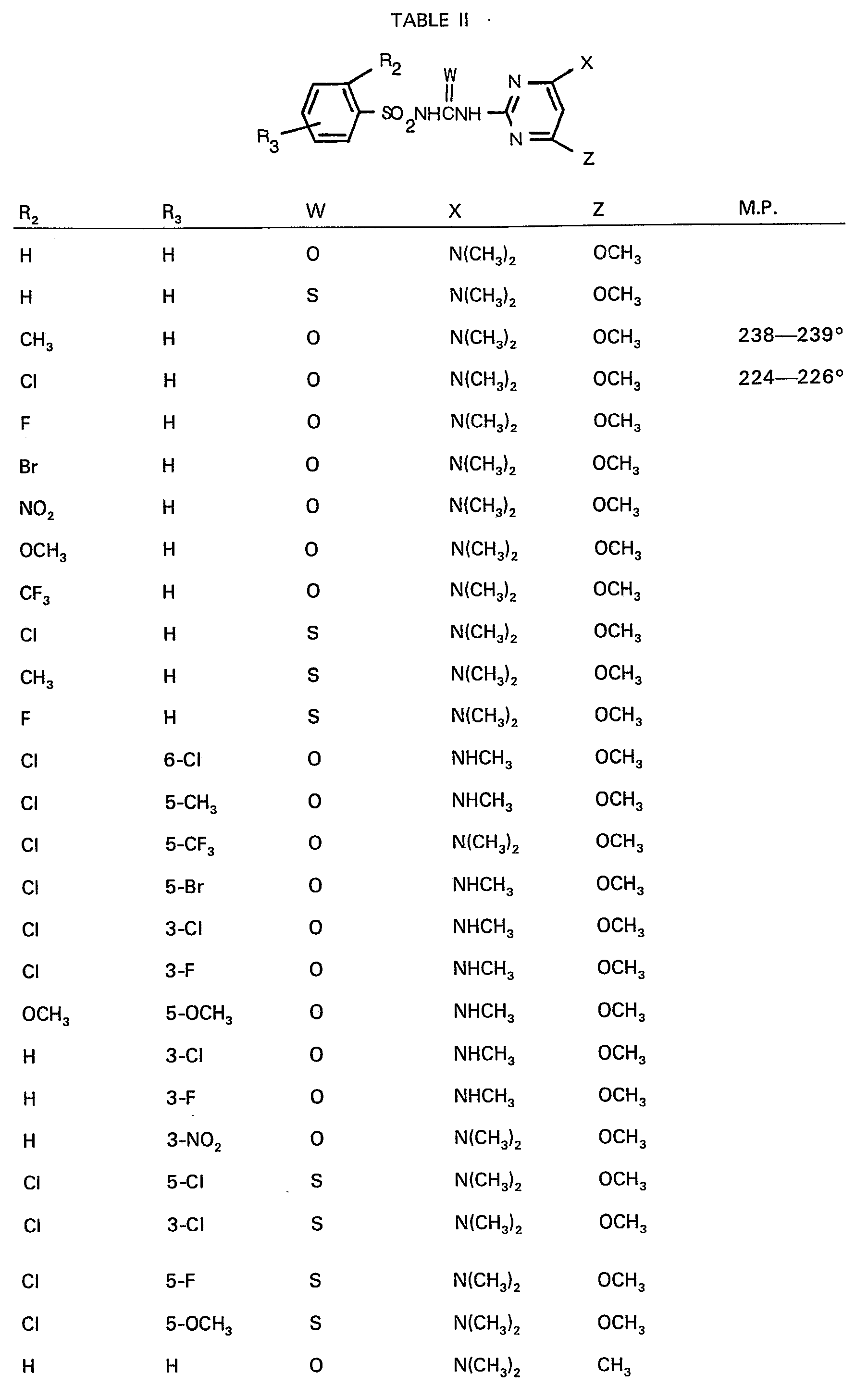

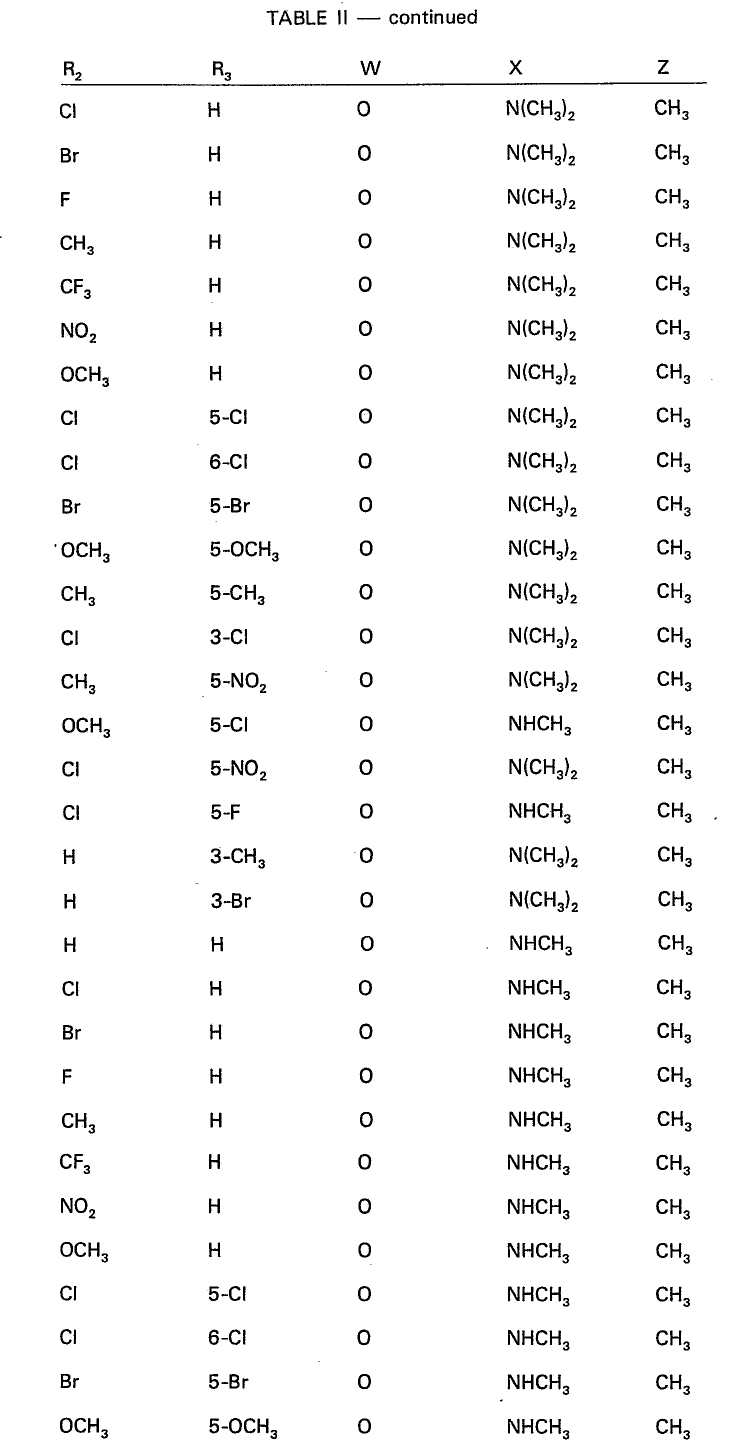

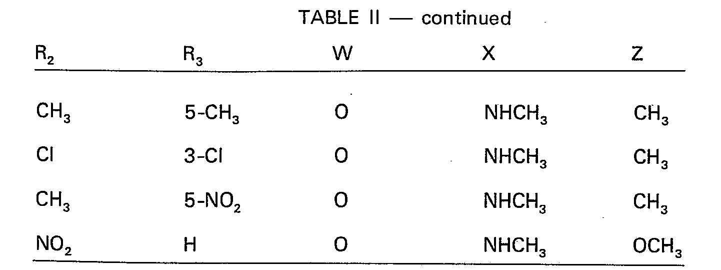

### Formulations

Useful formulations of the compounds of Formula I can be prepared in conventional ways. They include dusts, granules, pellets, solutions, suspensions, emulsions, wettable powders, emulsifiable concentrates and the like. Many of these may be applied directly. Sprayable formulations can be extended in suitable media and used at spray volumes of from a few liters to several hundred liters per hectare. High strength compositions are primarily used as intermediates for further formulations. The formulations, broadly, contain 0.1 % to 99% by weight of active ingredient(s) and at least one of a) 0.1 % to 20% surfactant(s) and b) 1% to 99.9% solid or liquid diluent(s). More specifically, they will contain these ingredients in the following approximate proportions:

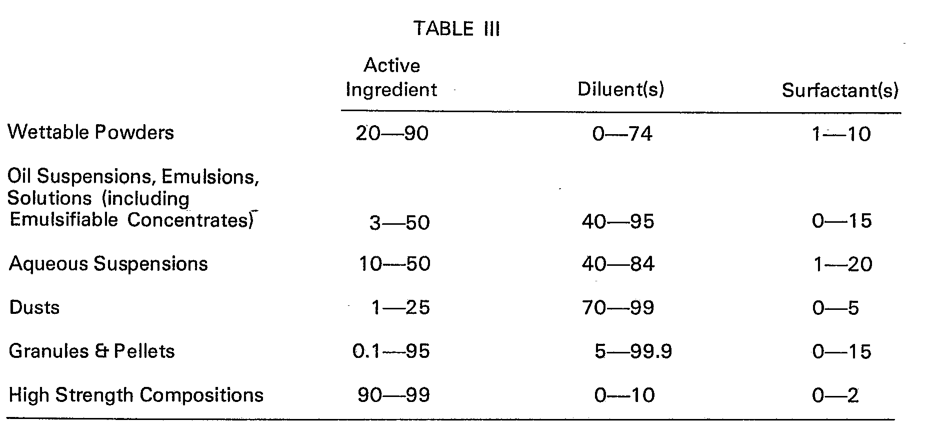

Lower or higher levels of active ingredient can, of course, be present depending on the intended use and the physical properties of the compound. Higher ratios of surfactant to active ingredient are sometimes desirable, and are achieved by incorporation into the formulation or by tank mixing.

Typical solid diluents are described in Watkins et al., "Handbook of Insecticide Dust Diluents and Carriers", 2nd Ed., Dorland Books, Caldwell, New Jersey. The more absorptive diluents are preferred for wettable powders and the denser ones for dusts. Typical liquid diluents and solvents are described in Marsden, "Solvents Guide", 2nd Ed., Interscience, New York (1950). Solubility under 0.1 % is preferred for suspension concentrates; solution concentrates are preferably stable against phase separation at 0°C. "McCutcheon's Detergents and Emulsifiers Annual", MC Publishing Corp., Ridgewood, New Jersey, as well as Sisely and Wood, "Encyclopedia of Surface Active Agents", Chemical Publishing Co., Inc., New York (1964), list surfactants and recommended uses. All formulations can contain minor amounts of additives to reduce foam, caking, corrosion, microbiological growth, etc.

The methods of making such compositions are well known. Solutions are prepared by simply mixing the ingredients. Fine solid compositions are made by blending and, usually, grinding as in a hammer or fluid energy mill. Suspensions are prepared by wet milling (see, for example, Littler, U.S. Patent 3,060,084). Granules and pellets may be made by spraying the active material upon preformed granular carriers or by agglomeration techniques. See J. E. Browning, "Agglomeration", Chemical Engineering, Dec. 4, 1967, pp. 147ff. and "Perry's Chemical Engineer's Handbook", 4th Ed., McGraw-Hill, New York (1963), pp. 8-59 ff.

For further information regarding the art of formulation, see for example:
H. M Loux, U.S. Patent 3,235,361, February 15, 1966, Col. 6, line 16 through Col. 7, line 19 and Examples 10 through 41.
R. W. Luckenbaugh, U.S. Patent 3,309,192, March 14, 1967, Col. 5, line 43 through Col. 7, line 62 and Examples 8, 12, 15, 39, 41, 52, 53, 58, 132, 138-140, 162-164, 166, 167, 169―182.
H. Gysin and E. Knusli, U.S. Patent 2,891,855, June 23, 1959, Col. 3, line 66 through Col. 5, line 17 and Examples 1―4.
G. C. Klingman, "Weed Control as a Science", John Wiley & Sons, Inc., New York (1961), pp. 81-96.
J. D. Fryer, and S. A. Evans, "Weed Control Handbook", 5th Ed., Blackwell Scientific Publications, Oxford, (1968), pp. 101-103.

In the following examples, all parts are by weight unless otherwise indicated.

### Example 7

### Wettable powder

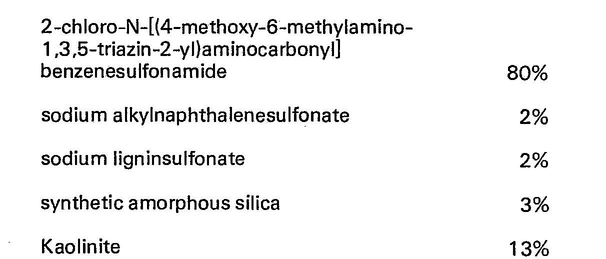

The ingredients are blended, hammer-milled until all the solids are essentially under 50 microns, reblended and packaged.

### Example 8

### Wettable Powder

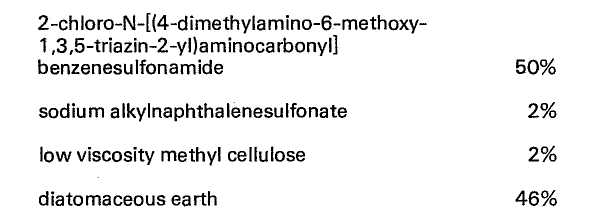

The ingredients are blended, coarsely hammer-milled and then air-milled to produce particles essentially all below 10 microns in diameter. The product is reblended before packaging.

### Example 9

### Granule

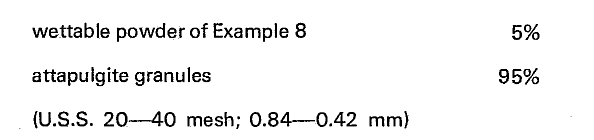

A slurry of wettable powder containing ≃25% solids is sprayed on the surface of attapulgite granules in a double-cone blender. The granules are dried and packaged.

### Example 10

### Extruded pellets

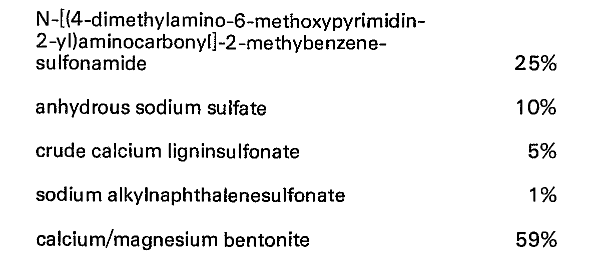

The ingredients are blended, hammer-milled and then moistened with about 12% water. The mixture is extruded as cylinders about 3 mm diameter which are cut to produce pellets about 3 mm long. These may be used directly after drying, or the dried pellets may be crushed to pass a U.S.S. No. 20 sieve (0.84 mm openings). The granules held on a U.S.S. No. 40 sieve (0.42 mm openings) may be packaged for use and the fines recycled.

### Example 11

### Oil Suspension

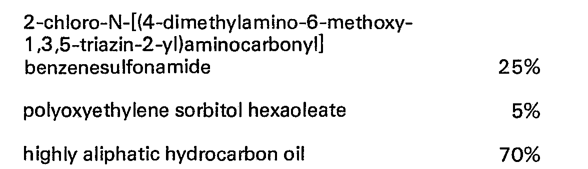

The ingredients are ground together in a sand mill until the solid particles have been reduced to under about 5 microns. The resulting suspension may be applied directly, but preferably after being extended with oils or emulsified in water.

### Example 12

### Wettable Powder

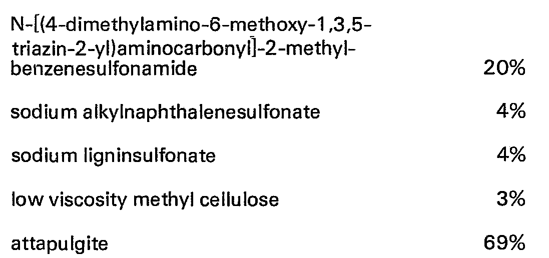

The ingredients are thoroughly blended. After grinding in a hammer mill to produce particles essentially all below 100 microns, the material is reblended and sifted through a U.S.S. No. 50 sieve (0.3 mm opening) and packaged.

### Example 13

### Oil Suspension

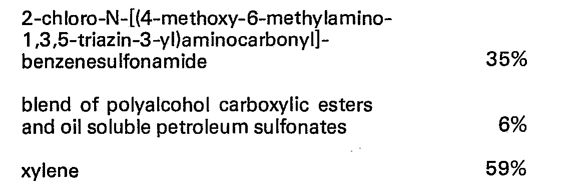

The ingredients are combined and ground together in a sand mill to produce particles essentially all below 3 microns. The product can be used directly, extended with oils, or emulsified in water.

### Example 14

### High Strength Concentrate

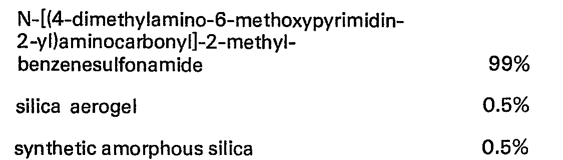

The ingredients are blended and ground in a hammer mill to produce a material essentially all passing a U.S.S. No. 50 screen (0.3 mm opening). The concentrate may be formulated further if necessary.

### Example 15

### Low Strength Granule

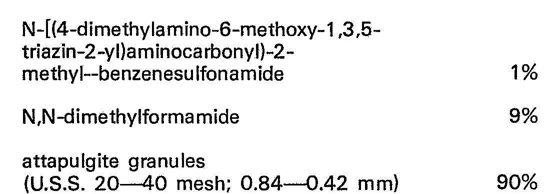

The active ingredient is dissolved in the solvent and the solution is sprayed upon dedusted granules in a rotating blender. After spraying of the solution has been completed, the blender is allowed to run for a short period and then the granules are packaged.

### Example 16

### Aqueous Suspension

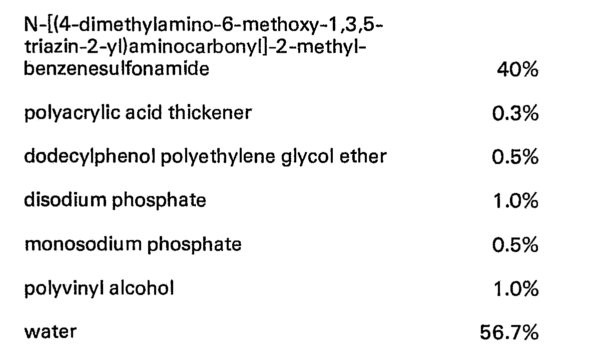

The ingredients are blended and ground together in a sand mill to produce particles essentially all under 5 microns in size.

### Example 17

### Solution -

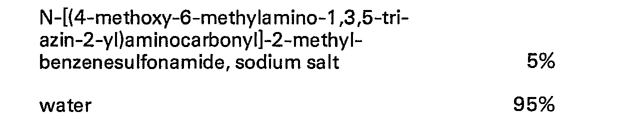

The salt is added directly to the water with stirring to produce the solution, which may then be packaged for spray use.

### Example 18

### Granule

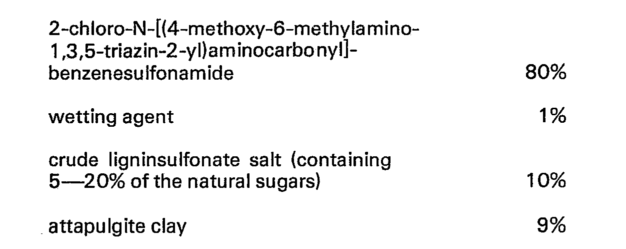

The ingredients are blended and milled to pass through a 100 mesh screen. The material is then added to a fluid bed granulator, the air flow is adjusted to gently fluidize the material, and a fine spray of water is sprayed onto the fluidized material. The fluidization and spraying are continued until granules of the desired size range are made. The spraying is stopped, but fluidization is continued, optionally with heat, until the water content is reduced to the desired level, generally less than 1 %. The material is then discharged, screened to the desired size range, generally 14-100 mesh (1410-149 microns), and packaged for use.

### Example 19

### Low Strength Granule

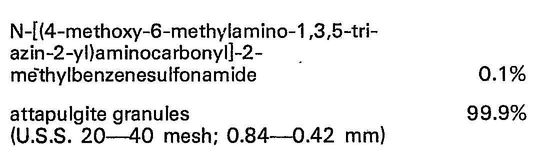

The active ingredient is dissolved in a solvent and the solution is sprayed upon dedusted granules is a double cone blender. After spraying of the solution has been completed, the material is warmed to evaporate the solvent. The material is allowed to cool and then packaged.

### Example 20

### Wettable Powder

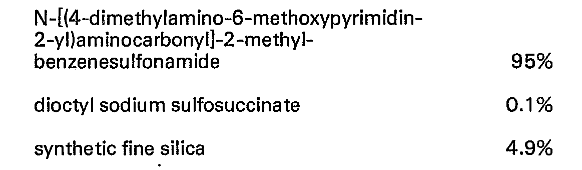

The ingredients are blended and ground in a hammer mill to produce particles essentially all below 100 microns. The material is sifted through a U.S.S. No. 50 screen (0.3 mm opening) and then packaged.

### Example 21

### Wettable Powder

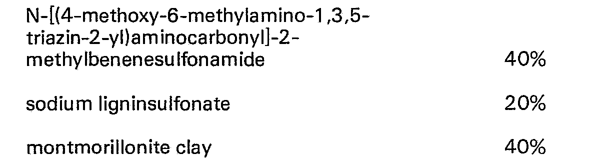

The ingredients are thoroughly blended, coarsely hammer-milled and then air milled to produce particles essentially all below 10 microns in size. The material is reblended and then packaged.

The compounds of Formula I can be formulated using the procedures of Examples 7-21.

### UTILITY

The compounds of Formule I are useful as herbicides. They may be applied either pre- or postemergence for the control of undesired vegetation in non-crop areas or the selective weed control in certain crops, e.g. wheat and soybeans. By properly selecting rate and time of application, compounds of this invention may be used to modify plant growth beneficially.

The precise amount of the compound of Formula I to be used in any given situation will vary according to the particular end result desired, the use involved, the weeds to be controlled, the soil type, the formulation and mode of application, weather conditions, etc. Since so many variables play a role, it is not possible to state a rate of application suitable for all situations. Broadly speaking, the compounds of this invention are used at levels of about 0.1 to 20 kg/ha with a preferred range of 0.1 to 10 kg/ha. The lower rates of the range will generally be selected for lighter soils, for selective weed control in crops, or in situations where maximum persistence is not necessary. Some of the compounds of Formula I can be used at very low rates for plant growth modification, but higher rates may also be useful, depending on factors such as the crop being treated, timing of treatment, etc.

The compounds of Formula I may be combined with other herbicides and are particularly useful in combination with 3-(3,4-dichlorophenyl)-1,1-dimethylurea, the triazines such as 2-chloro-4-(ethyl- amino)-6-(isopropylamino)-s-triazine, the uracils such as 5-bromo-3-sec-butyl-6-methyluracil, N-(phosphonomethyl)-glycine, 3-cyclohexyl-1-methyl-6-dimethylamino-s-triazine-2,4(1H,3H)-dione, N,N-dimethyl-2,2-diphenylacetamide, 2,4-dichlorophenoxyacetic acid (and closely related compounds), 4-chloro-2-butynyl-3-chlorophenylcarbamate, diisopropylthiolcarbamic acid, ester with 2,3-dichloroallyl alcohol, diisopropylthiolcarbamic acid, S-(2,3,3-trichloroallyl) ester, ethyl-N-benzoyl-N-(3,4-dichlorophenyl)-2-aminopropionate, 1,2-dimethyl-3,5-diphenylpyrazolium methylsulfate, methyl 2-[4-(2,4-dichlorophenoxy)-phenoxy]propanoate, 4-amino-6-tert-butyl-3-(methylthio)-1,2,4-triazin-5(4H)-one, 3-(3,4-dichlorophenyl)-1-methoxy-1-methylurea, 3-isopropy!-1H-2,1,3-benzothio- diazin-(4)-3H-one 2,2-dioxide, a,a,a-trifluoro-2,6-dinitro-N,N-dipropyl-p-toluidine, 1,1'-dimethyl-4,4'-bipyridinium ion, monosodium methanearsonate, 2-chloro-2',6'-diethyl(methoxymethyl) acetanilide, and 1,1-dimethyl-3-(α,α,α-trifluoro-m-tolyl)urea.

The activity of these compounds was discovered in greenhouse tests. The tests are described and the data resulting from them are shown below.

### Test Procedure

Seeds of crabgrass (Digitaria sp), baryardgrass (Echinochloa crusgall^{y}, wild oats (Avena fatua), Cassia tora, morningglory (Ipomoea sp.), cocklebur (Xanthium sp.), sorghum, corn, soybean, rice, wheat and nutsedge tubers were planted in a growth medium and treated preemergence with the chemicals dissolved in a nonphytotoxic solvent. At the same time, cotton having five leaves (including cotyledonary ones), bush beans with the third trifoliolate leaf expanding, crabgrass with two leaves, barnyardgrass with two leaves, wild oats with one leaf, cassia with three leaves (including cotyledonary ones), morningglory with four leaves (including the cotyledonary ones), cocklebur with four leaves (including the cotyledonary ones), sorghum with three leaves, corn with three leaves, soybean with two cotyledonary leaves, rice with two leaves, wheat with one leaf, and nutsedge with three-five leaves were sprayed. Treated plants and controls were maintained in a greenhouse for 16 days, then all species were compared to controls and visually rated for response to treatment, using 1-3 ratings in each test.

Ratings for compounds tested by this procedure are recorded in Table III.

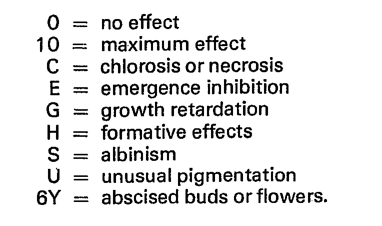

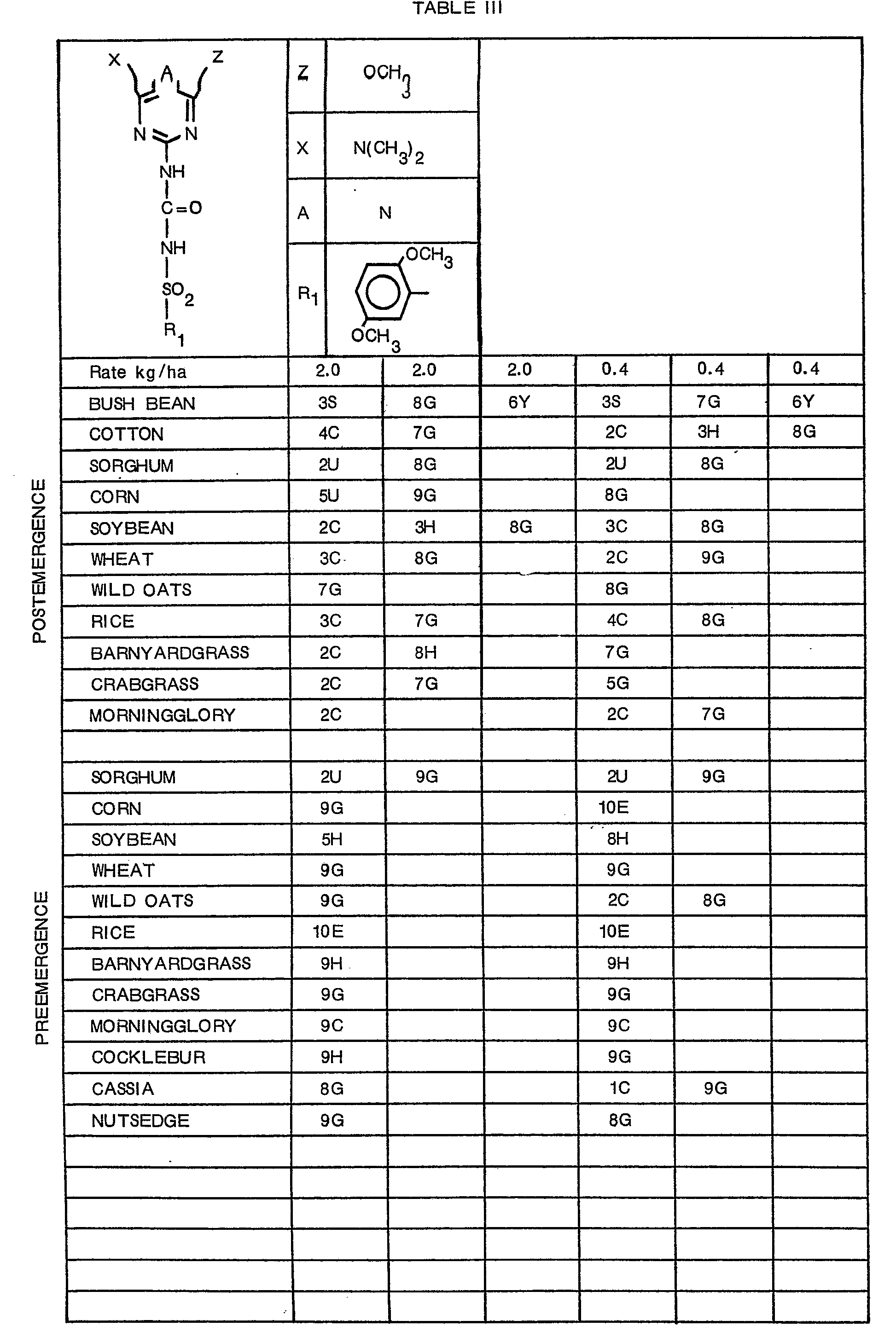

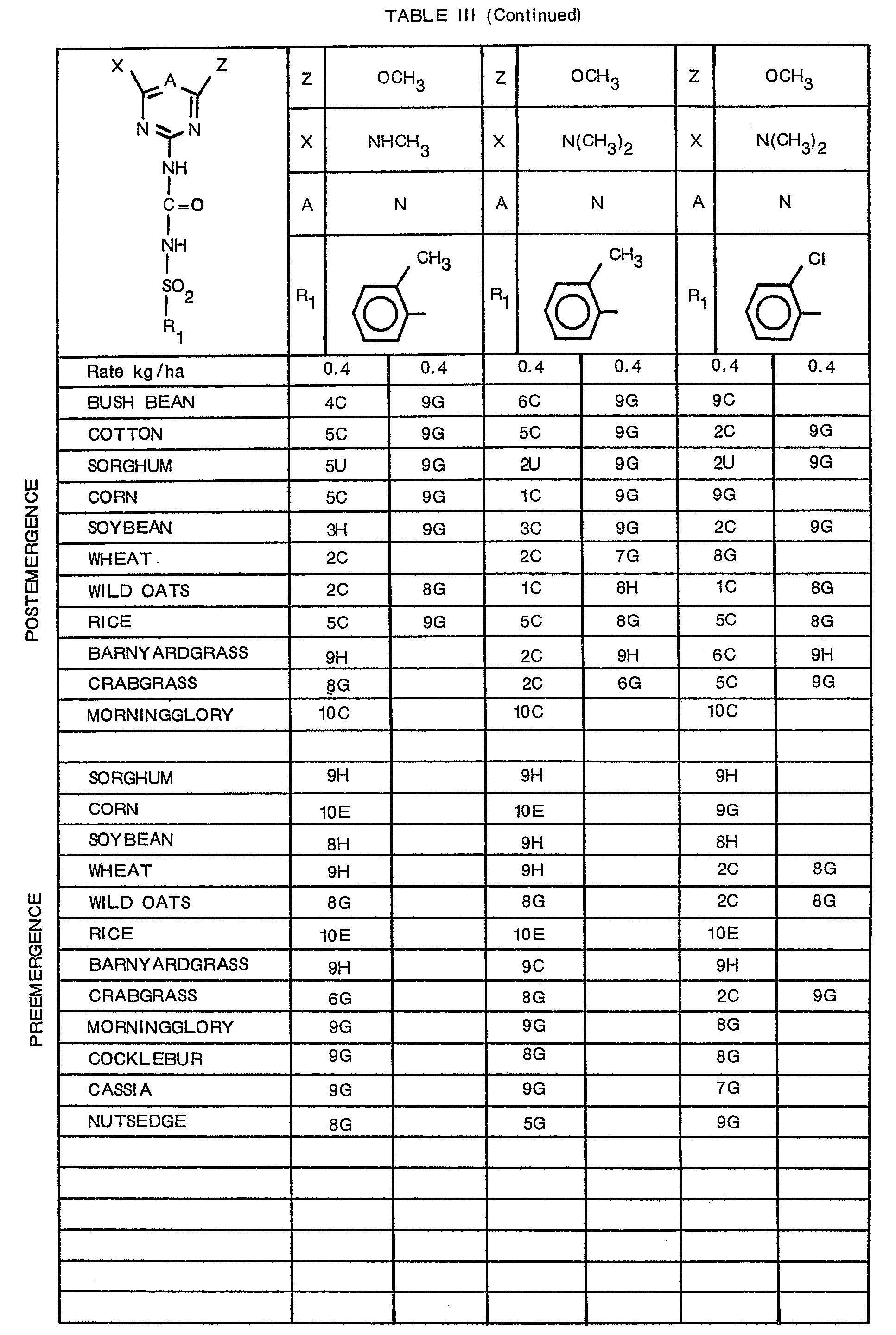

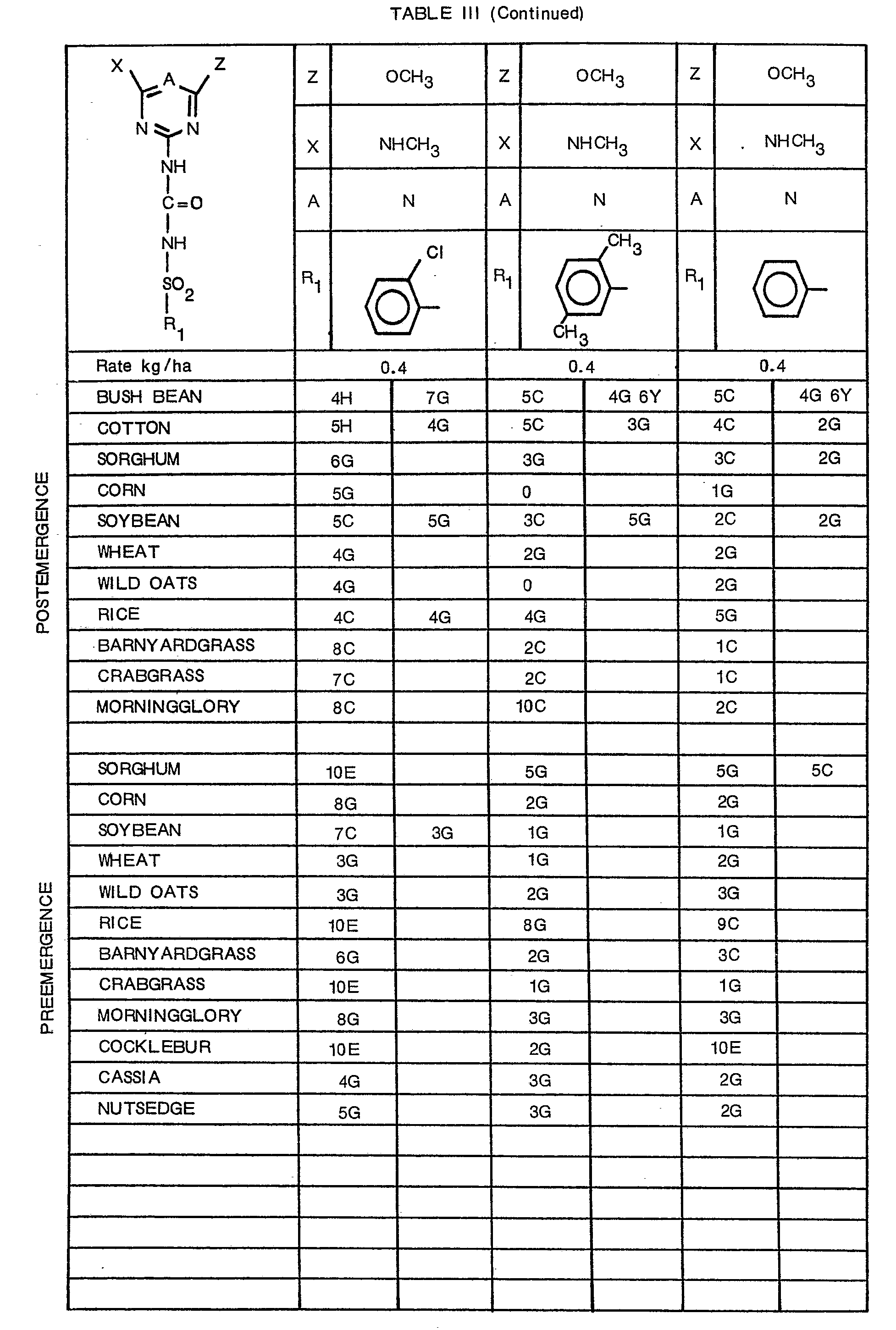

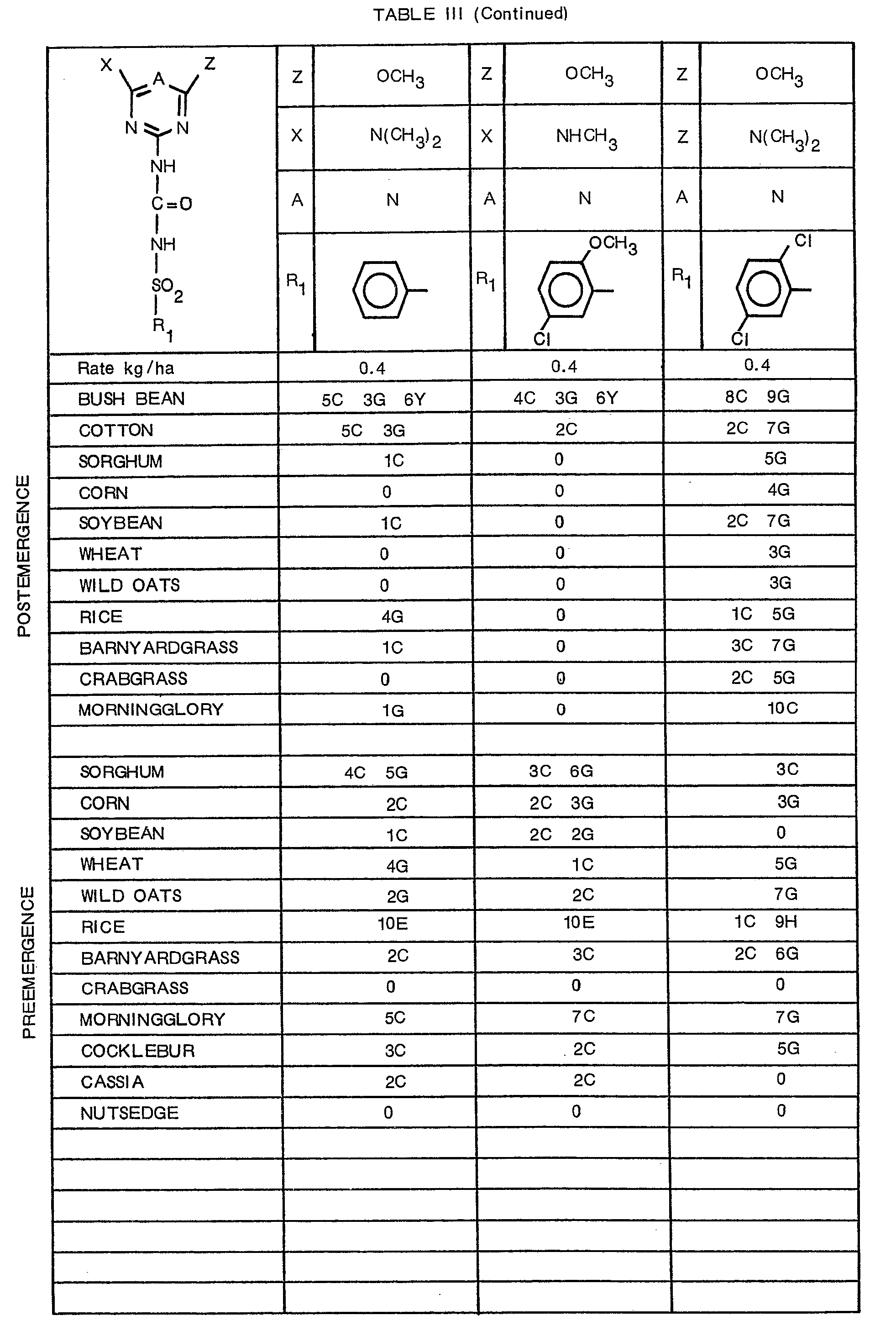

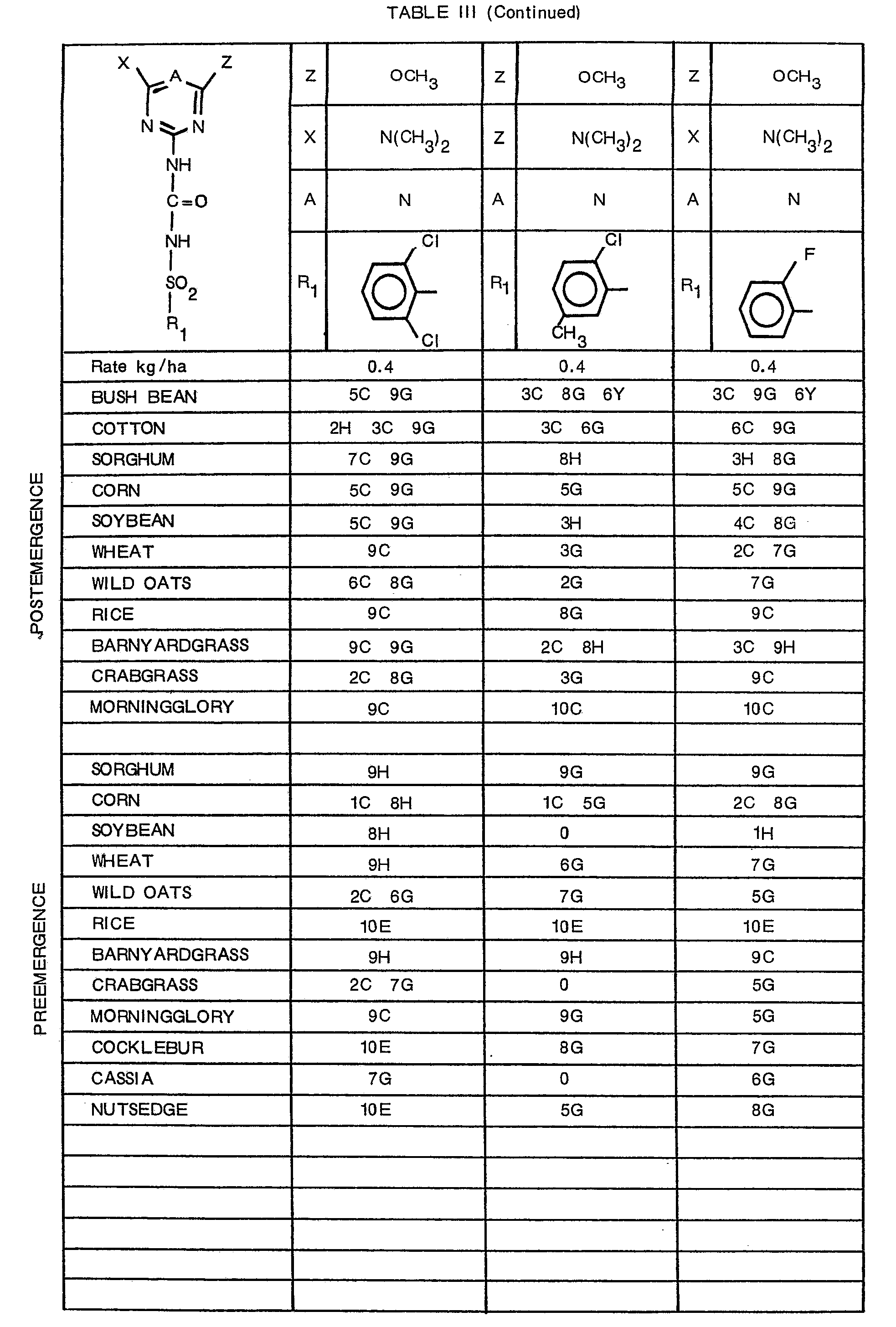

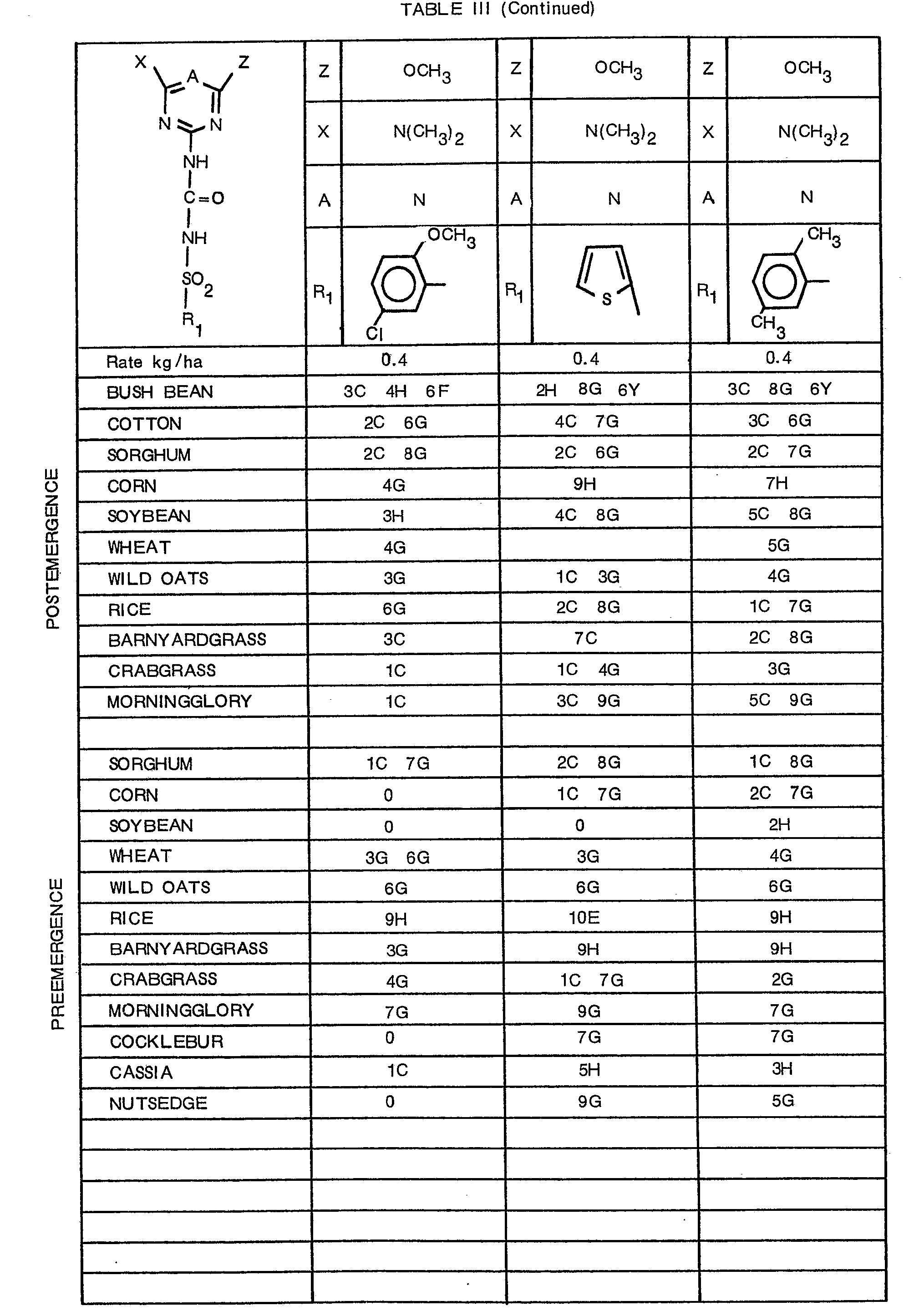

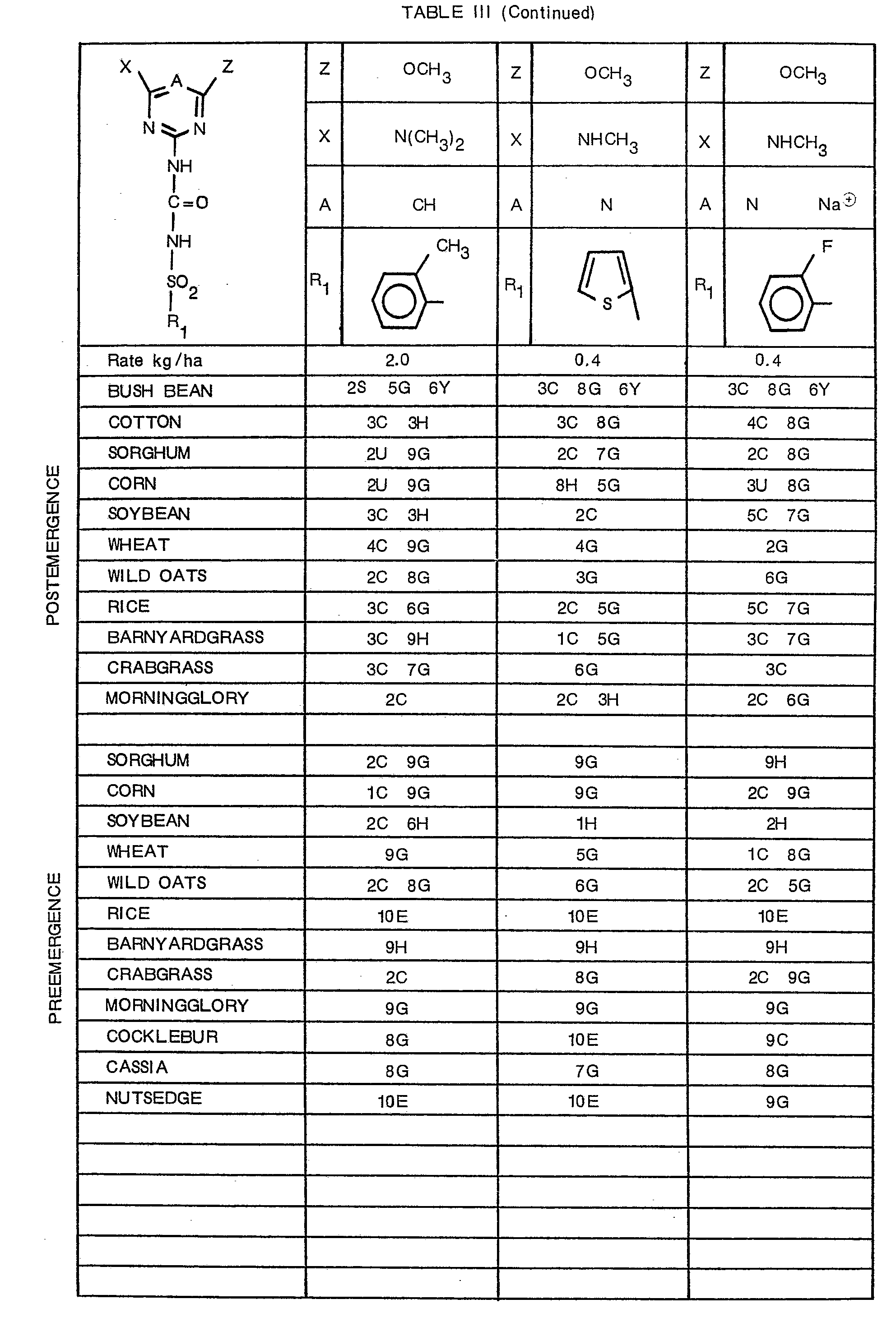

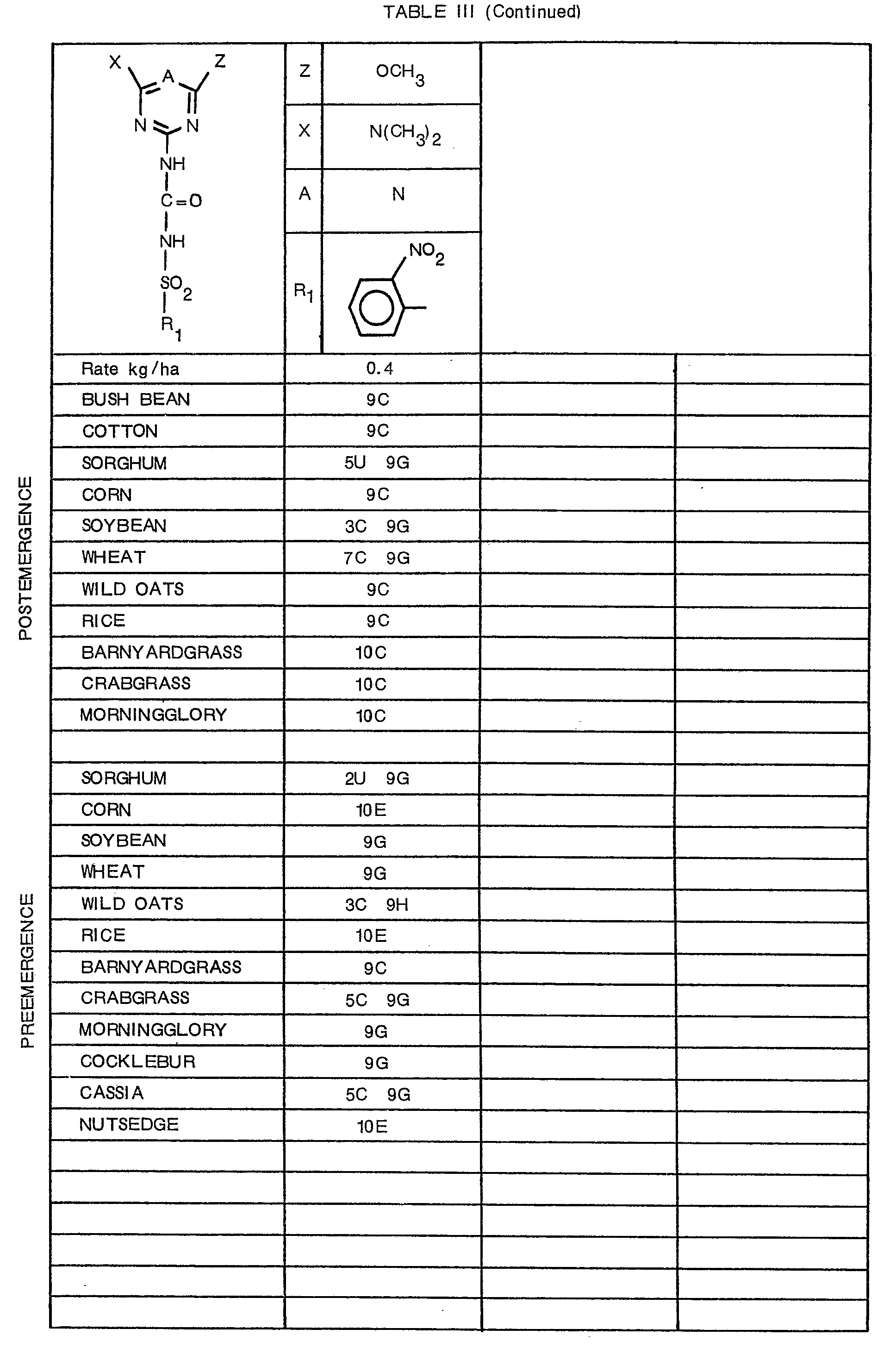

Utility of the compounds of the invention for selective weed control in wheat and soybeans was first observed in greenhouse tests. The test described below (B) illustrates these utilities.

### Test B

Two 25 cm diameter plastic bulb pans were filled with fertilized and limed Fallsington silt loam soil. One pan was planted with corn, sorghum and several grassy weeds. The other pan was planted with soybeans, purple nutsedge (Cyperus rotundus), and several broadleaf weeds. The following grassy and broadleaf species were planted: crabgrass (Digitaria sanguinalis), barnyardgrass (Echinochloa crusgalli), wild oats (Avena fatua), johnsongrass (Sorghum halepense), giant foxtail (Setaria faberii), Kentucky bluegrass (Poa pratensis), cheatgrass (Bromus secalinus), mustard (Brassica arvensis), cocklebur (Xanthium pennsylvanicum), pigweed (Amaranthus retroflexus), curly indigo (Aeschynomene virginica), morningglory (Ipomoea hederacea), cassia (Cassia tora), teaweed (Sida spinosa), velvetleaf (Abutilon theophrasti), and jimsonweed (Datura stramonium). In addition, two 12.5 cm diameter paper cups were filled with prepared soil; one was planted with rice and wheat, the other with sugarbeets. The above four containers were treated preemergence, i.e., the compounds were sprayed on the soil surface before seed germination.

Twenty-eight days after treatment, the plants were evaluated. The data obtained are summarized in Table IV. It should be noted that wheat has more tolerance for the compounds tested than most weed species.

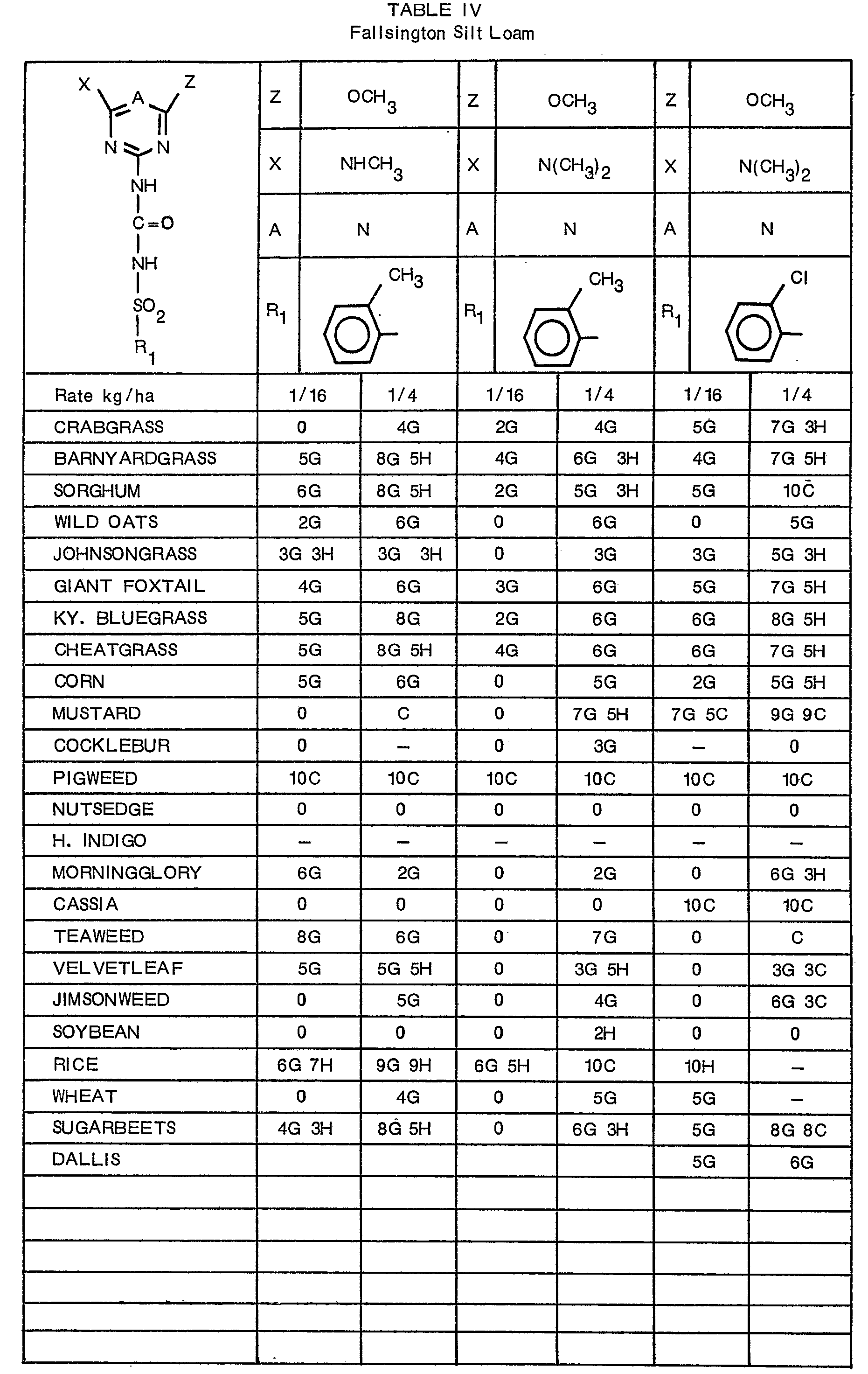

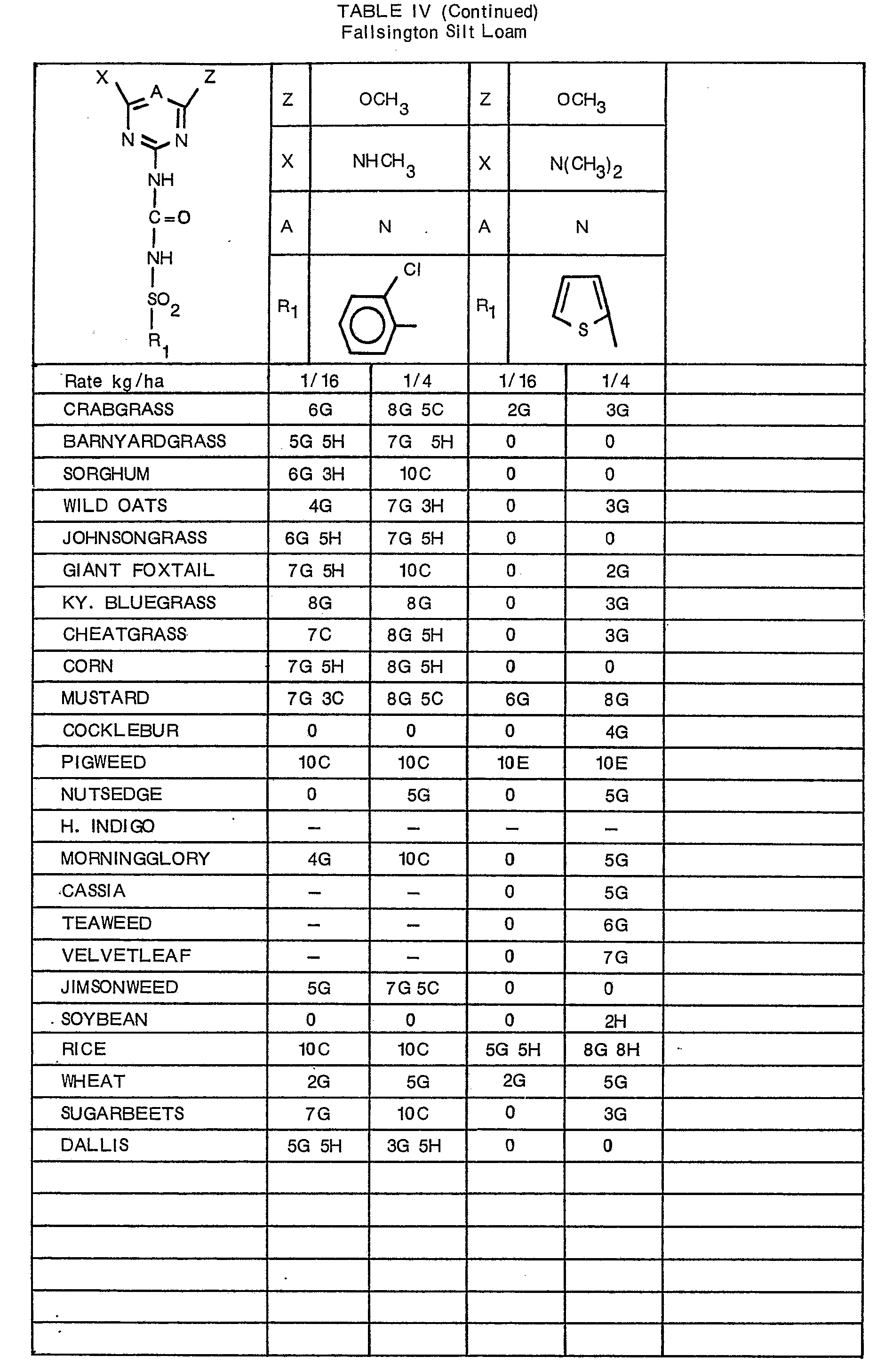

### Tect C

Twenty-five cm - diameter plastic pots filled with Fallsington silt loam were planted with soybean, cotton, alfalfa, corn, rice, wheat, sorghum, velvetleaf (Abutilon theophrastri), sesbania (Sesbania exaltata), cassia (Cassia tora), morningglory (Ipomoea sp.), jimsonweed (Datura stramonium), cocklebur (Xanthium pennsylvanicum), crabgrass (Digitaria sp.), nutsedge (Cyperus rotundus), barnyardgrass (Echinochloa crusgalli), giant foxtail ( Setaria faberii), and wild oats (Avena fatua). Approximately 2-1/2 weeks after planting, the young plants and the soil around them were sprayed overall with the test chemicals dissolved in a nonphytotoxic solvent. Fourteen days after treatment, all species were compared to untreated controls and visually rated for response to treatment. The data are presented in Table V.

This test illustrates the utility of the compounds as general postemergence herbicides.

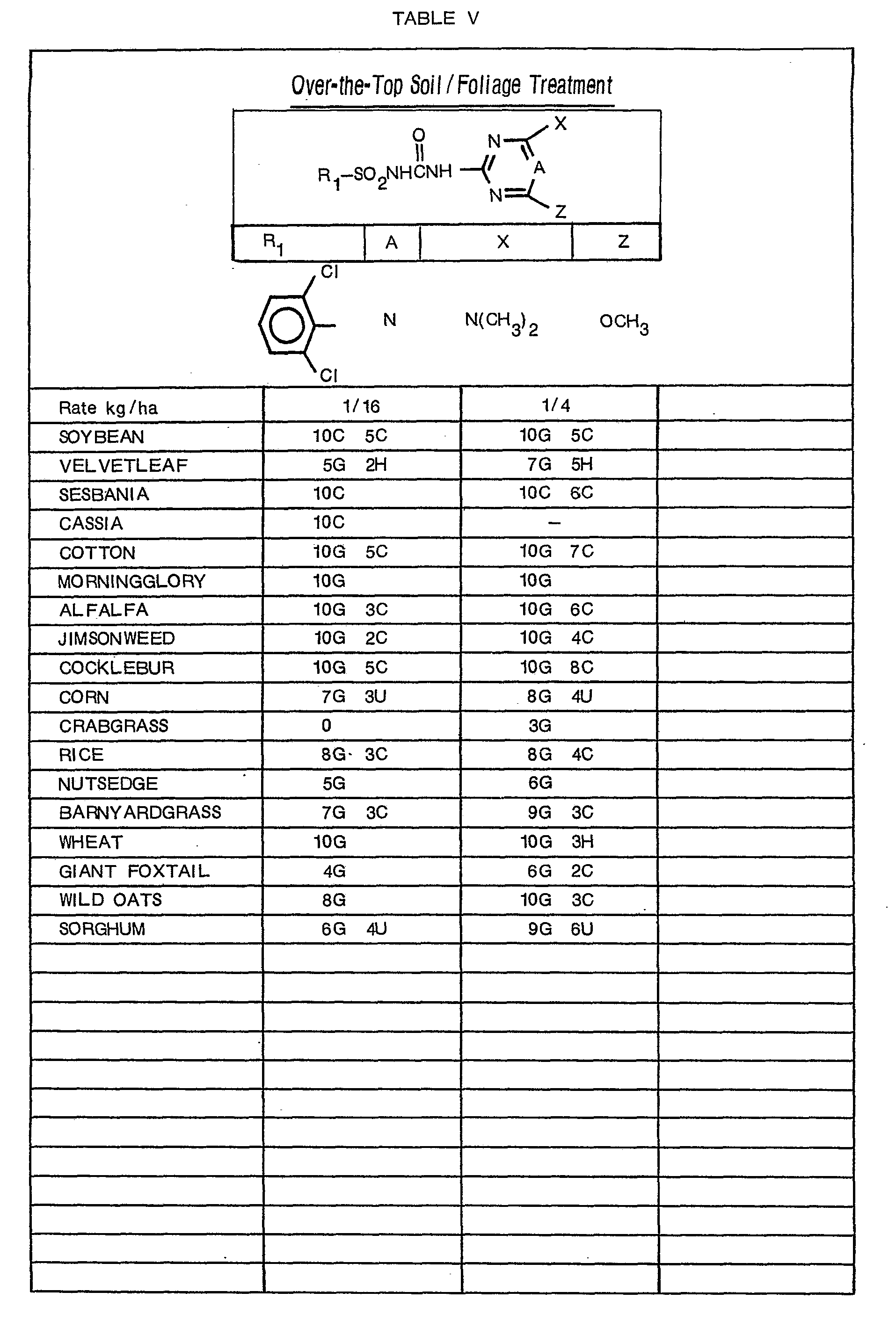

## Claims

1. A compound of general formula 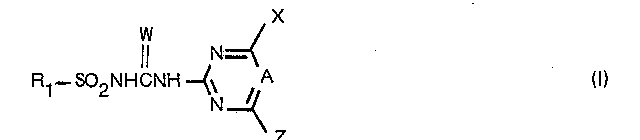 wherein 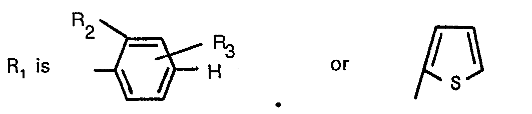
R₂ and R₃ are independently hydrogen, fluorine, chlorine, bromine, methyl, methoxy, nitro or trifluoromethyl;
W is oxygen or sulfur;
X is -NHCH₃ or -N(CH₃)_{z};
Z is methyl or methoxy; and
A is C or N H
or its agriculturally suitable salts, provided that when R₂ is nitro or trifluoromethyl, R₃ cannot be nitro or trifluoromethyl.

2. A compound of claim 1 wherein R, is

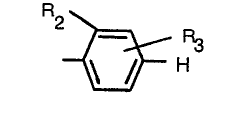

3. A compound of claim 1 or 2 wherein R₂ is fluorine, chlorine, bromine, methyl, or nitro.

4. A compound of any of claims 1-3, wherein R₃ is hydrogen, fluorine, chlorine, bromine, methyl, or nitro.

5. A compound of claim 2 wherein R₂ is chlorine, methyl or nitro; and R₃ is hydrogen, chlorine or methyl.

6. A compound of any of claims 1-5 wherein W is oxygen.

7. 2 - Chloro - N - [(4 - methoxy - 6 methylamino - 1,3,5 - triazin - 2 - yl)aminocarbonyl] - benzenesulfonamide or its agriculturally suitable salts.

8. 2 - Chloro - N - [(4 - dimethylamino - 6 - methoxy - 1,3,5 - triazin - 2 - yl)aminocarbonyl] - benzenesulfonamide or its agriculturally suitable salts.

9. N - [(4 - Dimethylamino - 6 - methoxy - 1,3,5 - triazin - 2 - yl)aminocarbonyl] - 2 - methylbenzenesulfonamide or its agriculturally suitable salts.

10. N - [(4 - Methoxy - 6 - methylamino - 1,3,5 - triazin - 2 - yl)aminocarbonyl] - 2 - methylbenzenesulfonamide or its agriculturally suitable salts.

11. N - [(4 - Dimethylamino - 6 - methoxypyrimidin - 2 - yl)aminocarbonyl] - 2 - methyl- benzenesulfonamide or its agriculturally suitable salts.

12. N - [(4 - Methylamino - 6 - methoxy - 1,3,5 - triazin - 2 - yl)aminocarbonyl] - 2 - nitrobenzenesulfonamide or its agriculturally suitable salts.

13. N - [(4 - Dimethylamino - 6 - methoxy - 1,3,5 - triazin - 2 - yl)aminocarbonyl] - 2 - nitrobenzenesulfonamide or its agriculturally suitable salts.

14. A composition for the control of undesirable vegetation comprising herbicide and at least one of (a) a surface active agent and (b) a solid or liquid diluent, characterised in that said herbicide is a compound of any of claims 1-13.

15. A method for the control of undesirable vegetation by applying to the locus of such undesirable vegetation an effective amount of a herbicidal compound characterised in that said herbicidal compound is a compound of any of claims 1-13.

16. A method for the selective control of undesirable vegetation in wheat by applying to the locus of such undesirable vegetation an effective amount of a herbicidal compound characterised in that said herbicidal compound is a compound of any of claims 1-13.

17. A method for the control of volunteer corn in soybean by applying to the locus of such volunteer corn an effective amount of a herbicidal compound characterised in that said herbicidal compound is a compound of any of claims 1-13.

18. A method for regulating the growth of crop plants by applying thereto an effective amount of a plant growth regulant characterised in that said plant growth regulant is a compound of any of claims 1-13.

19. A process for the preparation of a compound of claim 1 which comprises reacting a sulfonyl isocyanate or isothiocyanate of general formula 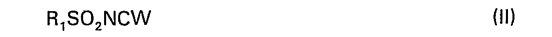 with a compound of general formula 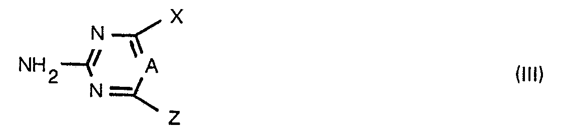 wherein R₁, W, X, Z and A are as defined in claim 1.

## Patentansprüche

1. Verbindungen der allgemeinen Formel 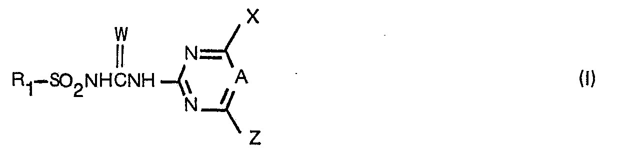 in der 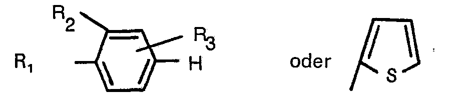 bedeutet,
R₂ und R₃ unabhängig voneinander Wasserstoff, Fluor, Chlor, Brom, Methyl, Methoxy, Nitro oder Trifluormethyl bedeuten,
W Sauerstoff oder Schwefel bedeutet,
X -NHCH₃ oder ―N(H₃)₂, bedeutet,
Z Methyl oder Methoxy bedeutet und
A C oder N bedeutet H
oder deren für landwirtschaftliche Zwecke geeignete Salze, mit der Massgabe, dass, wenn R₂ Nitro oder Trifluormethyl bedeutet, R₃ nicht Nitro oder Trifluormethyl sein kann.

2. Verbindungen nach Anspruch 1, dadurch gekennzeichnet, dass R₁ 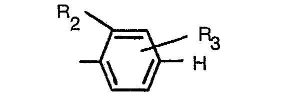 bedeutet.

3. Verbindungen nach Anspruch 1 oder 2, dadurch gekennzeichnet, dass R₂ Fluor, Chlor, Brom, Methyl, oder Nitro bedeutet.

4. Verbindungen nach einem der Ansprüche 1 bis 3, dadurch gekennzeichnet, dass R₃ Wasserstoff, Fluor, Chlor, Brom, Methyl oder Nitro bedeutet.

5. Verbindungen nach Anspruch 2, dadurch gekennzeichnet, dass R₂ Chlor, Methyl oder Nitro bedeutet und R₃ Wasserstoff, Chlor oder Methyl bedeutet.

6. Verbindungen nach einem der Ansprüche 1 bis 5, dadurch gekennzeichnet, dass W Sauerstoff bedeutet.

7. 2 - Chlor - N - [(4 - methoxy - 6 - methylamino - 1,3,5 - triazin - 2 - yl) - aminocarbonyl] - benzolsulfonamid oder für landwirtschaftliche Zwecke geeignete Salze davon.

8. 2 - Chlor - N - [(4 - dimethylamino - 6 - methoxy - 1,3,5 - triazin - 2 - yl) - aminocarbonyl] - benzolsulfonamid oder für landwirtschaftliche Zwecke geeignete Salze davon.

9. N - [(4 - Dimethylamino - 6 - methoxy - 1,3,5 - triazin - 2 - yl) - aminocarbonyl] - 2 - methylbenzolsulfonamid oder für landwirtschaftliche Zwecke geeignete Salze davon.

10. N - [(4 - Methoxy - 6 - methylamino - 1,3,5 - triazin - 2 - yl) - aminocarbonyl] - 2 - methylbenzolsulfonamid oder für landwirtschaftliche Zwecke geeignete Salze davon.

11. N - [(4 - Dimethylamino - 6 - methoxypyrimidin - 2 - yl) - aminocarbonyl] - 2 - methylbenzolsulfonamid oder für landwirtschaftliche Zwecke geeignete Salze davon.

12. N - [(4 - Methylamino - 6 - methoxy - 1,3,5 - triazin - 2 - yl) - aminocarbonyl] - 2 - nitrobenzolsulfonamid oder für landwirtschaftliche Zwecke geeignete Salze davon.

13. N - [(4 - Dimethylamino - 6 - methoxy - 1,3,5 - triazin - 2 - yl) - aminocarbonyl] - 2 - nitrobenzolsulfonamid oder für landwirtschaftliche Zwecke geeignete Salze davon.

14. Zusammensetzungen zur Kontrolle von unerwünschter Vegetation, enthaltend ein Herbizid und mindestens (a) ein oberflächenaktives Mittel und (b) ein festes oder flüssiges Verdünnungsmittel, dadurch gekennzeichnet, dass es sich bei dem Herbizid um eine Verbindung nach einem der Ansprüche 1 bis 13 handelt.

15. Verfahren zur Kontrolle von unerwünschter Vegetation durch Aufbringen einer wirksamen Menge einer herbiziden Verbindung auf die Stelle der unerwünschten Vegetation, dadurch gekennzeichnet, dass es sich bei der herbiziden Verbindung um eine Verbindung nach einem der Ansprüche 1 bis 13 handelt.

16. Verfahren zur selektiven Kontrolle von unerwünschter Vegetation in Weizen durch Aufbringen einer wirksamen Menge einer herbiziden Verbindung auf die Stelle der unerwünschten Vegetation, dadurch gekennzeichnet, dass es sich bei der herbiziden Verbindung um eine Verbindung nach einem der Ansprüche 1 bis 13 handelt.

17. Verfahren zur Kontrolle von wildwachsendem Getreide in Sojabohnen durch Aufbringen einer wirksamen Menge einer herbiziden Verbindung auf die Stelle des wildwachsenden Getreides, dadurch gekennzeichnet, dass es sich bei der herbiziden Verbindung um eine Verbindung nach einem der Ansprüche 1 bis 13 handelt.

18. Verfahren zur Regelung des Wachstums von Kulturpflanzen durch Aufbringen einer wirksamen Menge eines Pflanzenwuchsreglers, dadurch gekennzeichnet, dass es sich beim Pflanzenwuchsregler um eine Verbindung nach einem der Ansprüche 1 bis 13 handelt.

19. Verfahren zur Herstellung der Verbindungen nach Anspruch 1, dadurch gekennzeichnet, dass man ein Sulfonylisocyanat oder Isothiocyanat der allgemeinen Formel 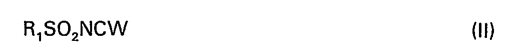 mit einer Verbindung der allgemeinen Formel 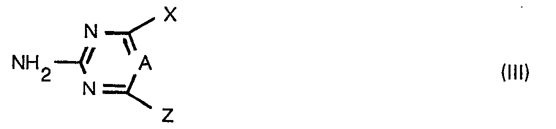 umsetzt, wobei R₁, W, X, Z und A die in Anspruch 1 definierte Bedeutung haben.

## Revendications

1. Un composé de la formule générale 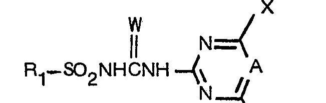 dans laquelle 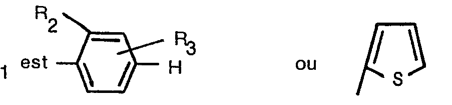
R₂ et R₃ sont indépendamment l'hydrogène, le fluor, le chlore, le brome, le groupe méthyle, méthoxy, nitro ou trifluorométhyle;
W est de l'oxygène ou du soufre;
X est -NHCH₃ ou -N(CH₃)₂;
Z est un groupe méthyle ou méthoxy; et
A est C ou N H
ou ses sels utilisables en agriculture, avec la condition que, quand R₂ est un groupe nitro ou trifluorométhyle, R₃ ne peut pas être un groupe nitro ou trifluorométhyle.

2. Un composé selon la revendication 1 dans lequel R₁ est

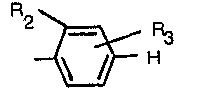

3. Un composé selon l'une des revendications 1 ou 2, dans lequel R₂ est du fluor, du chlore, du brome, un groupe méthyle ou nitro.

4. Un composé selon l'une quelconque des revendications 1 à 3, dans lequel R₃ est de l'hydrogène, du fluor, du chlore, du brome, un groupe méthyle ou nitro.

5. Un composé selon la revendication 2 dans lequel R₂ est du chlore, un groupe méthyle ou nitro et R₃ est de l'hydrogène, du chlore ou un groupe méthyle.

6. Un composé selon l'une quelconque des revendications 1 à 5 dans lequel W est de l'oxygène.

7. Le 2 - chloro - N - [(4 - méthoxy - 6 - méthylamino - 1,3,5 - triazin - 2 - yl)aminocarbonyl] - benzènesulfonamide ou ses sels utilisables en agriculture.

8. Le 2 - chloro - N - [(4 - diméthylamino - 6 - méthoxy - 1,3,5 - triazin - 2 - yl)aminocarbonyl] - benzènesulfonamide ou ses sels utilisables en agriculture.

9. Le N - [(4 - diméthylamino - 6 - méthoxy - 1,3,5 - triazin - 2 - yl)aminocarbonyl] - 2 - méthylbenzènesulfonamide ou ses sels utilisables en agriculture.

10. Le N - [(4 - méthoxy - 6 - méthylamino - 1,3,5 - triazin - 2 - yl)aminocarbonyl] - 2 - méthylbenzènesulfonamide ou ses sels utilisables en agriculture.

11. Le N - [(4 - diméthylamino - 6 - méthoxypyrimidin - 2 - yllaminocarbonyl] - 2 - méthyl- benzènesulfonamide ou ses sels utilisables en agriculture.

12. Le N - [(4 - méthylamino - 6 - méthoxy - 1,3,5 - triazin - 2 - yl)aminocarbonyl] - 2 - nitrobenzènesulfonamide ou ses sels utilisables en agriculture.

13. Le N - [(4 - diméthylamino - 6 - méthoxy - 1,3,5 - triazin - 2 yl)aminocarbonyl] - 2 - nitrobenzènesulfonamide où ses sels utilisables en agriculture.

14. Une composition pour lutter contre la végétation indésirable comprenant un herbicide et au moins un ingrédient choisi parmi (a) un agent tensio-actif et (b) un diluant solide ou liquide, caractérisée en ce que l'herbicide est un composé selon l'une quelconque des revendications 1 à 13.

15. Une méthode de lutte contre la végétation indésirable en appliquant au site de cette végétation indésirable une quantité efficace d'un composé herbicide, caractérisée en ce que le composé herbicide est un composé selon l'une quelconque des revendications 1 à 13. -

16. Une méthode de lutte sélective contre la végétation indésirable dans le blé en appliquant au site de cette végétation indésirable une quantité efficace d'un composé herbicide, caractérisé en ce que le composé herbicide est un composé selon l'une quelconque des revendications 1 à 13.

17. Une méthode de lutte contre le maïs accidentel dans le soja en appliquant au site de ce maïs accidentel une quantité efficace d'un composé herbicide, caractérisé en ce que le composé herbicide est un composé selon l'une quelconque des revendications 1 à 13.

18. Une méthode de régulation de la croissance de plantes cultivées en appliquant à ces plantes une quantité efficace d'un agent de rétulation de la croissance des plantes, caractérisé en ce que l'agent de régulation de la croissance des plantes est un composé selon l'une quelconque des revendications 1 à 13.

19. Un procédé de préparation d'un composé tel que défini dans la revendication 1, selon lequel on fait réagir un isocyanate ou isothiocyanate de sulfonyle de la formule générale 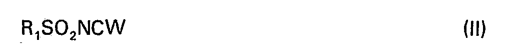 avec un composé de la formule générale 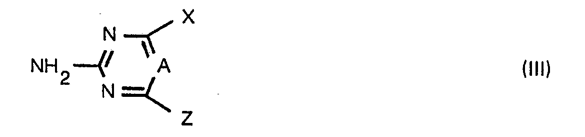 où R₁, W, X, Z et A sont tels que définis dans la revendication 1.
